# EUROPEAN PATENT APPLICATION

(11) **EP 3 108 884 A1**
(43) Date of publication of application: **28.12.2016**
(21) Application number: 15752334.1
(22) Date of filing: 16.02.2015
(51) Int. Cl.: A61K 31/22, A61K 41/00, A61K 45/00, A61P 35/00, A61P 43/00

(54) **COMPOSITION FOR PREVENTION OR TREATMENT OF TREATMENT-RESISTANT CANCER**

(30) Priority: 21.02.2014 JP 2014031220
(71) Applicant: SBI Pharmaceuticals Co., Ltd., Tokyo 106-6020 (JP); Osaka Medical and Pharmaceutical College, Takatsuki-shi Osaka 5698686 (JP)
(72) Inventor: ISHIKAWA, Toshihisa, Yokohama-shi Kanagawa 226-0016 (JP); KUROIWA, Toshihiko, Takatsuki-shi Osaka 569-8686 (JP); KAJIMOTO, Yoshinaga, Takatsuki-shi Osaka 569-8686 (JP); FUJISHIRO, Takahiro, Takatsuki-shi Osaka 569-8686 (JP); KIMURA, Seigo, Takatsuki-shi Osaka 569-8686 (JP); MIYATAKE, Shin-Ichi, Takatsuki-shi Osaka 569-8686 (JP); TANAKA, Tohru, Tokyo 106-6020 (JP)
(74) Representative: Weickmann & Weickmann PartmbB
(86) International application number: PCT/JP2015/054134
(87) International publication number: WO 2015/125732

(57) **Abstract**

[Problem to be Solved] It is an object of the present invention to provide a preventive or therapeutic composition for a therapy-resistant cancer having therapy-resistant cancer cells.

[Solution] A preventive or therapeutic composition for a therapy-resistant cancer having therapy-resistant cancer cells, which is for use in photodynamic therapy, wherein the composition comprises ALAs.

## Description

### [Technical Field]

The present invention relates to a preventive or therapeutic composition for a therapy-resistant cancer having therapy-resistant cancer cells used in photodynamic therapy (PDT), etc.

### [Background Art]

One of choices for cancer therapy, other than anticancer drug therapy and radiation therapy, is photodynamic therapy (PDT). Since PDT is a therapeutic method which is non-invasive and hardly causes treatment scars, it has recently attracted attention.

An example of the PDT which has been currently practiced in clinical sites is a therapeutic method, which comprises administering to an early cancer developed in the lung, esophagus, stomach or uterine cervix, photofrin as a tumor-affinity photosensitizer reacting with a laser having a specific wavelength, accumulating the photofrin in cancer cells, and applying laser beam to the cancer cells, so as to destroy the cancer cells from the inside thereof. However, in such a therapeutic method, since photofrin has a low excretion speed and thus easily accumulated in a body, a patient develops photosensitive dermatitis due to room light or other lights. Hence, this therapeutic method is problematic in that a patient must stay in a dark room until the photosensitizer is excreted from the body (for approximately several weeks to approximately one month after completion of the administration).

In contrast, what is called "ALA-PDT" has been known, and the ALA-PDT comprises administering to a subject, 5-aminolevulinic acids (which are also referred to as "ALAs" in the present description), which are precursors of porphyrin and are biological substances, and combining the ALAs with PDT. Safe and efficient photodynamic therapy for cancer, which involves administration of ALAs, has been studied.

It has been known that ALAs themselves do not have photosensitivity, but the ALAs are metabolically activated in cells by a series of enzymes in a heme biosynthetic pathway so that they are converted to porphyrins (which are mainly protoporphyrin IX (PpIX)). In addition, such PpIX has been known as a photosensitizer having peaks at 410 nm, 510 nm, 545 nm, 580 nm, 630 nm, etc. Since ALA is metabolized in a living body and is then excreted from the body within 48 hours, the ALA is characterized in that it has almost no influences on the systemic photosensitivity. Thus, ALA has higher clinical safety than photofrin.

So far, when a surgical operation is performed on malignant brain tumor, ALA has been administered to a subject to perform photodynamic diagnosis (PDD) (what is called "ALA-PDD").

On the other hand, PDT performed on malignant brain tumor, which involves administration of ALA, has not yet been carried out in clinical sites. One of the reasons is a decrease in PpIX accumulation due to the excretion of the PpIX from malignant brain tumor cells. In addition, there has been another problem that the boundary between cancer tissues and normal tissues surrounding the cancer tissues becomes unclear due to PpIX excreted from the cancer cells, and a portion in which tumor has infiltrated into normal brain tissues cannot be excised, so that the success rate of excision of a malignant brain tumor by a surgical operation is reduced.

Moreover, except for surgically resectable early cancers, complete cure of cancer has been still a great challenge. A majority of cancers for which surgical operation is difficult or undesirable are treated by anticancer drug therapy or radiation therapy. However, it is extremely difficult to completely eliminate tumor cells. As such, in many cases, cancer cells that have acquired therapy resistance (resistant cancer cells) would appear.

Furthermore, in recent years, cancer stem cell hypothesis has been proposed as a cause of recurrence and/or metastasis. According to such cancer stem cell hypothesis, it is considered that cancer stem cells are present in tumor tissues, and that the cancer stem cells have self-replication ability and also have an ability to form the same tumor as the original tumor tissues, even if the number of the cancer stem cells is small. Further, since such cancer stem cells have resistance to anticancer agents or radiation, they easily remain after the treatment, and thus, these cells are considered to become a cause of recurrence and/or metastasis.

Accordingly, although a majority of cancer cells have been eliminated by the conventional cancer therapy, cancer cells are generated again from the aforementioned therapy-resistant cancer cells (cancer stem cells, resistant cancer cells, etc.), which remain after completion of the treatment. Thus, these therapy-resistant cancer cells can be a cause of cancer metastasis and cancer recurrence. That is to say, in the conventional cancer therapy, even in a case where cancer cells have died as a result of cancer therapy and a patient is considered to recover from the cancer, only a small number of therapy-resistant cancer cells still remain in many cases, and thus, the conventional cancer therapy could not be a radical treatment of cancer.

Therefore, it has been desired for a long time in the society to establish a therapeutic method that targets therapy-resistant cancer cells, so as to realize cancer therapy having a low risk of recurrence and metastasis.

However, ALA-PDT and ALA-PDD, which are applied to therapy-resistant cancer cells, have not been studied so far, and the effectiveness thereof has not been known.

### [Citation List]

### [Patent Literature]

[Patent Literature 1] Japanese Patent Laid-Open No. 11-12197
[Patent Literature 2] National Publication of International Patent Application No. 1999-501914

### [Summary of Invention]

### [Technical Problem]

It is an object of the present invention to provide a preventive or therapeutic composition for a therapy-resistant cancer having therapy-resistant cancer cells.

### [Solution to Problem]

The present inventors have focused on ALA-PDT having an action mechanism on cells, which is different from that of anticancer drug therapy or radiation therapy. As a result of intensive studies, the present inventors have surprisingly discovered that ALA-PDT is effective for therapy-resistant cancer cells.

Moreover, the present inventors have found that therapy-resistant cancer cells can be more efficiently killed by performing ALA-PDT on the therapy-resistant cancer cells multiple times (multiple stages). That is to say, the inventors have found that, when ALA-PDT is carried out multiple times, therapy-resistant cancer cells, which have remained after a first stage of ALA-PDT, can be completely killed, for example, by a second or later stage of ALA-PDT.

Subsequently, the present inventors have not only achieved an extremely important object in the medical field, as described above, but they have also tried to achieve a further important object. Specifically, the present inventors have conceived of a case where sufficient effects on therapy-resistant cancer cells cannot be obtained even by utilizing ALA-PDT. For example, the inventors have focused on the possibility that, in a deep part of a solid tumor or the like, a light cannot reach cancer cells as therapeutic targets, and thus sufficient therapeutic effects cannot be obtained. Furthermore, even if a method involving a light capable of reaching a deep part of a tumor is applied, this method cannot be considered to be a radical treatment of cancer, if it cannot completely kill therapy-resistant cancer cells.

Regarding this matter, it has also been elucidated that since PpIX that has been biosynthesized from ALA is excreted from cancer cells, accumulation of the PpIX serving as a photosensitizer in cancer cells is suppressed, and thus, a decrease in the PDT efficiency to the cancer tissues occurs.

As such, in order to achieve these important objects, the present inventors have focused on promotion of the accumulation of PpIX in cells, and have continued a trial-and-error approach. As a result, the inventors have discovered that the expression of the transporters PEPT1 and PEPT2, which are associated with incorporation of ALA in cells, the expression of porphyrin synthase or an ABCB6 gene, and the like, are accelerated in therapy-resistant cancer cells. In addition, the present inventors have discovered that a tyrosine kinase inhibitor inhibits ABCG2 associated with the excretion of PpIX in therapy-resistant cancer cells. Based on these findings, the present inventors have found that accumulation of PpIX in cells is improved by inhibition of ABCG2, and the effects of ALA-PDT on therapy-resistant cancer cells can be thereby enhanced. That is, the present inventors have focused on the action mechanism of PpIX, and have found a compound capable of enhancing the therapeutic effects of ALA-PDT on therapy-resistant cancer cells, and thereby, the inventors have also realized further inhibition of the growth of therapy-resistant cancer cells and/or the death thereof.

Further, the present inventors have surprisingly found that the boundary between cancer tissues and normal tissues can be clarified by using a drug capable of inhibiting ABCG2 (preferably, a tyrosine kinase inhibitor) in combination with ALAs, and that the cancer tissues comprising therapy-resistant cancer cells can be then detected based on the fluorescence of PpIX accumulated in cancer cells.

(1) Specifically, in one embodiment, the present invention relates to a preventive or therapeutic composition for a therapy-resistant cancer having therapy-resistant cancer cells used in photodynamic therapy, comprising a compound represented by the following formula (I), or a salt thereof:
   [Formula 1] 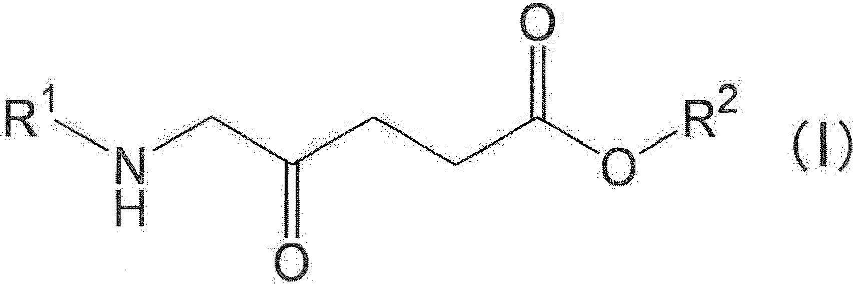 wherein R¹ represents a hydrogen atom or an acyl group, and R² represents a hydrogen atom, a linear or branched alkyl group, a cycloalkyl group, an aryl group, or an aralkyl group.
(2) In addition, in one embodiment, the present invention relates to the above described preventive or therapeutic composition, wherein the therapy-resistant cancer cells comprise cancer stem cells.
(3) Moreover, in one embodiment, the present invention relates to the above described preventive or therapeutic composition, wherein the cancer stem cells are brain tumor stem cells.
(4) Furthermore, in one embodiment, the present invention relates to the above described preventive or therapeutic composition, wherein the photodynamic therapy is carried out on therapy-resistant cancer cells in a subject two or more times.
(5) Further, in another embodiment, the present invention relates to a preventive or therapeutic medicament comprising a combination of (i) the above-mentioned preventive or therapeutic composition and (ii) an anticancer agent administered simultaneously or sequentially.
(6) Further, in one embodiment, the present invention relates to the above described preventive or therapeutic medicament, wherein the anticancer agent is a tyrosine kinase inhibitor.
(7) Further, in one embodiment, the present invention relates to the above described preventive or therapeutic medicament, wherein
   the tyrosine kinase inhibitor is a tyrosine kinase inhibitor acting against a molecule having a tyrosine residue, and
   the molecule having a tyrosine residue is selected from the group consisting of a stem cell factor receptor (KIT), an epidermal growth factor receptor (EGFR), a nerve growth factor receptor (NGFR), a colony-stimulating factor receptor (CSF-1R), a hepatocyte growth factor receptor (HGFR), a fibroblast growth factor receptor (FGFR), a vascular endothelial growth factor receptor (VEGFR), a platelet-derived growth factor receptor (PDGFR), a human epidermal growth factor receptor 2 (HER2/neu), a Src family, JAK, Fak, ZAP, Btk, Fps/Fes, and Bcr-Abl.
(8) Still further, in one embodiment, the present invention relates to the above described preventive or therapeutic medicament, wherein the tyrosine kinase inhibitor is an ABCG2 inhibitor.
(9) Still further, in one embodiment, the present invention relates to the above described preventive or therapeutic medicament, wherein the combination aspect is a combination drug or a kit.
(10) Still further, in another embodiment, the present invention relates to the above described preventive or therapeutic composition, used in combination simultaneously or sequentially with an anticancer agent.
(11) In addition, in another embodiment, the present invention relates to a combination of (i) the above-mentioned preventive or therapeutic composition and (ii) an anticancer agent for treating a therapy-resistant cancer, wherein
   (i) the therapeutic composition and (ii) the anticancer agent are administered simultaneously or sequentially.
(12) Moreover, in another embodiment, the present invention relates to a method for preventing or treating a therapy-resistant cancer having therapy-resistant cancer cells in a subject, comprising:
   (I) a step of administering to a subject affected with or in danger of being affected with a therapy-resistant cancer the compound represented by the above described formula (I) or a salt thereof; and
   (II) a step of performing photodynamic therapy on the cancer cells in the subject.
(13) Furthermore, in one embodiment, the present invention relates to the above described preventive or therapeutic method, wherein, in the step (II), the photodynamic therapy is performed on the therapy-resistant cancer cells two or more times.
(14) Further, in one embodiment, the present invention relates to the above described preventive or therapeutic method, further comprising a step of administering an anticancer agent simultaneously or sequentially to the subject, before, during, or after the step (I) or (II).
(15) Further, in another embodiment, the present invention relates to use of a compound represented by the above described (I), or a salt thereof, for production of a preventive or therapeutic medicament for a therapy-resistant cancer used in photodynamic therapy.
(16) Further, in another embodiment, the present invention relates to a medicament for diagnosing a therapy-resistant cancer having therapy-resistant cancer cells in photodynamic therapy, comprising a combination of:
   (A) the compound represented by the above described formula (I), or a salt thereof, and
   (B) a tyrosine kinase inhibitor or an ABCG2 inhibitor, administered simultaneously or sequentially.
(17) An invention made by arbitrarily combining the aforementioned one or multiple characteristics of the present invention is naturally included in the scope of the present invention, unless it includes a technical contradiction.

### [Advantageous Effects of Invention]

In one aspect, by using the preventive or therapeutic composition of the present invention in ALA-PDT, it becomes possible to prevent or treat a therapy-resistant cancer. In addition, in some cases, by performing ALA-PDT multiple times (multiple stages), the therapy-resistant cancer can be treated or prevented further efficiently.

Moreover, in another aspect, in some cases, by using the preventive or therapeutic composition of the present invention, simultaneously or sequentially with an anticancer agent, and preferably, with a tyrosine kinase inhibitor, the effects of ALA-PDT on a therapy-resistant cancer can be further enhanced.

Furthermore, in another aspect, since the present invention is able to reduce a risk of developing therapy-resistant cancer cells in the treatment process, or is able to kill cancer stem cells originally having therapy resistance, it is also effective for what is called a refractory cancer, and, it is also able to carry out radical cancer therapy that is not attended with the recurrence and metastasis of cancer. Further, by killing therapy-resistant cancer cells that cause the recurrence and metastasis of cancer, the present invention is also able to prevent the recurrence and metastasis of cancer.

Further, in another aspect, according to the present invention, by using a drug capable of inhibiting ABCG2 (preferably, a tyrosine kinase inhibitor) together with ALAs in ALA-PDD, the boundary between cancer tissues and normal tissues is clarified, and cancer tissues comprising therapy-resistant cancer cells can be easily excised by a surgical technique, based on the fluorescence of PpIX accumulated in the cancer tissues.

That is, the present invention is an extremely important invention in the medical field.

### [Brief Description of Drawings]

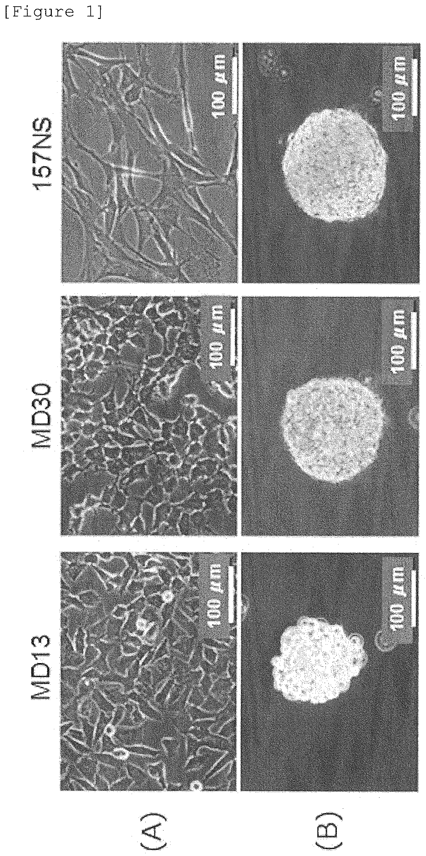

[Figure 1] Figure 1 is a view showing micrographs of brain tumor stem cells (MD13, MD30, and 157NS cells).
Figure 1A shows photographs regarding the culture of the cells with fetal calf serum (FCS) (non-sphere type).
Figure 1B shows photographs regarding the culture of the cells in a culture solution containing no FCS (sphere type).

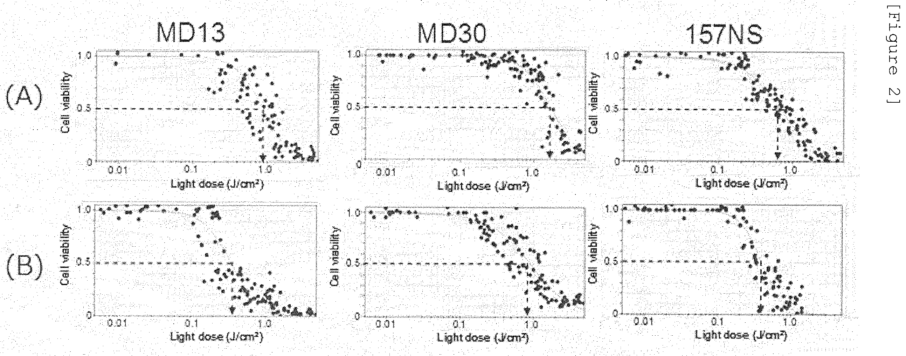

[Figure 2] Figure 2 is a view showing the sensitivity of brain tumor stem cells (MD13, MD30, and 157NS cells) to ALA-PDT. The MD13, MD30, and 157NS cells were cultured at a density of 1.0 x 10⁶ cells/ml, were then incubated with 0.3 mM ALA for 4 hours, and were then irradiated with 405-nm laser light. The intensity of the laser light was measured, and the relationship between the laser light intensity and the cell viability was plotted. Figure 2A is a (dot) plotting showing the cells that were cultured with FCS (non-sphere type). Figure 2B is a (dot) plotting showing the cells that were cultured in a culture solution containing no FCS (sphere type). The light intensity that reduces the cell viability to 50% (IC₅₀) is indicated with the arrow.

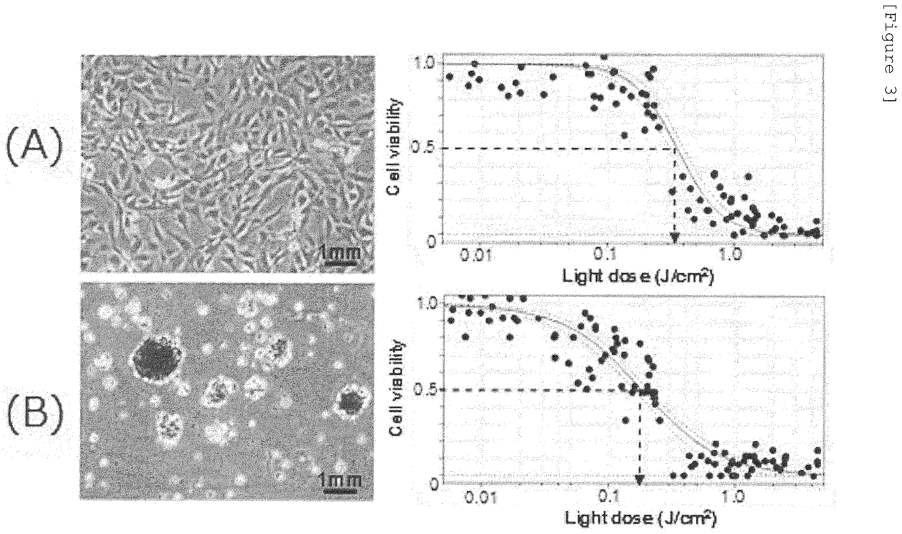

[Figure 3] Figure 3 is a view showing micrographs of brain tumor cells (A172 cells) and the photosensitivity of the cells to ALA-PDT. Figure 3A is a view showing the culture of the cells with FCS. Figure 3B is a view showing the culture of the cells without FCS, and the cells formed a mass (sphere) characteristic for stem cells. A172 cells were cultured at a density of 1.0 x 10⁶ cells/ml, were then incubated with 0.3 mM ALA for 4 hours, and were then irradiated with 450-nm laser light. The intensity of the laser light was measured, and the relationship between the laser light intensity and the cell viability was plotted. The light intensity that reduces the cell viability to 50% (IC₅₀) is indicated with the arrow.

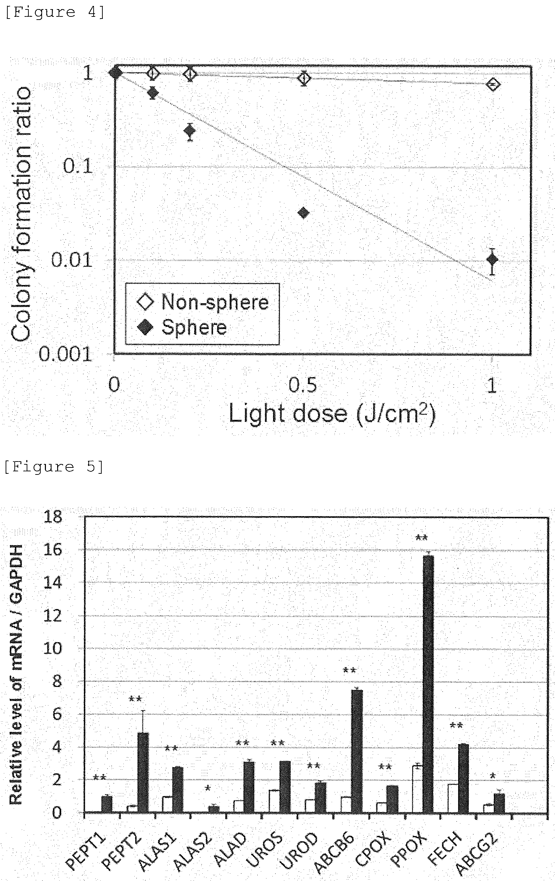

[Figure 4] Figure 4 is a view showing the photosensitivity of brain tumor cells (A172 cells) to ALA-PDT, from the viewpoint of cell proliferation ability. The A172 cells were cultured at a density of 1.0 x 10⁶ cells/ml, were then incubated with 0.3 mM ALA for 4 hours, and were then irradiated with 405-nm laser light. Thereafter, the cell proliferation ability of the cells with respect to light dose (J/cm²) was examined as colony-forming ability.
[Figure 5] Figure 5 is a view showing the mRNA levels of enzymes and transporters associated with the biosynthesis of porphyrin in brain tumor cells (A172 cells). Using the mRNA level of a Glyceraldehyde-3-phosphate dehydrogenase (GAPDH) gene as a control, the mRNA level of each gene was normalized.
   The measured genes: peptide transporters 1 and 2 (PEPT1 and PEPT2), 5-aminolevulinate synthases 1 and 2 (ALAS1 and ALAS2), 5-aminolevulinate dehydratase (ALAD), uroporphyrinogen III synthase (UROS), uroporphyrinogen decarboxylase (UROD), coproporphyrinogen oxidase (CPOX), protoporphyrinogen oxidase (PPOX), ferrochelatase (FECH), and ABC transporters (ABCB6 and ABCG2).

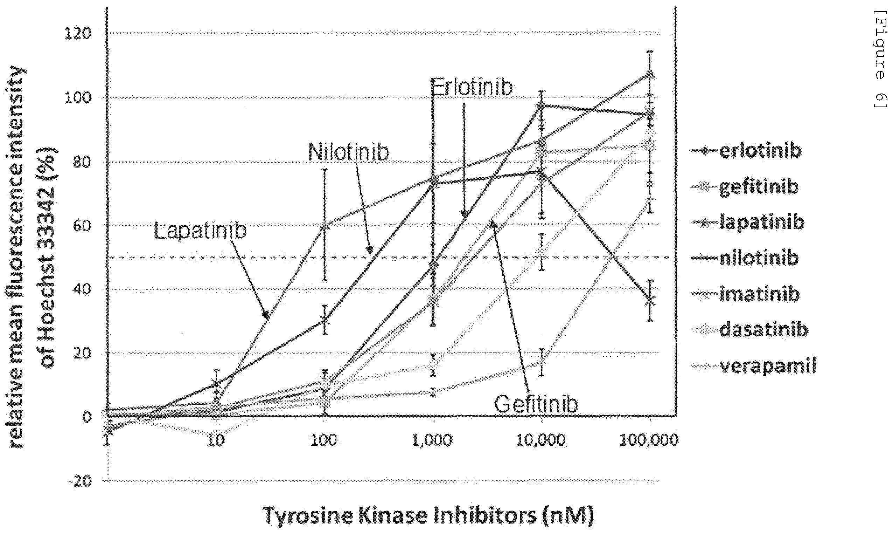

[Figure 6] Figure 6 is a view showing screening results regarding inhibition of the transport function of ABCG2 by tyrosine kinase inhibitors. Using, as ABCG2 inhibitors, Erlotinib, Gefitinib, Lapatinib, Imatinib, Nilotinib and Dasatinib, and also using Verapamil as a control, inhibition of the transport function of ABCG2 was screened. The concentration of the inhibitor was set in the range from 0 to 100 µM, and the extracellular transport of Hoechst 33342 by ABCG2 was measured.

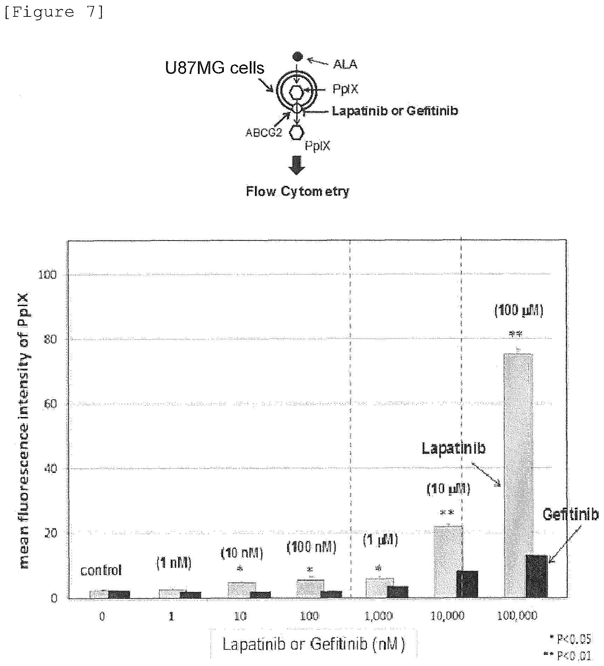

[Figure 7] Figure 7 is a view showing the influence of Lapatinib or Gefitinib on the intracellular accumulation of PpIX. The upper view is a schematic view of the assay, and the lower view shows the results thereof.

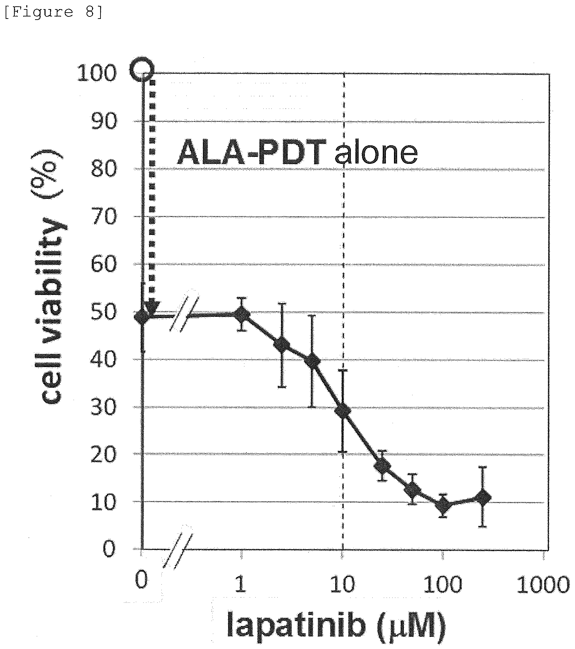

[Figure 8] Figure 8 is a view showing that the effects of ALA-PDT have been increased by addition of Lapatinib in an experiment using human glioblastoma astrocytoma U87MG cells.

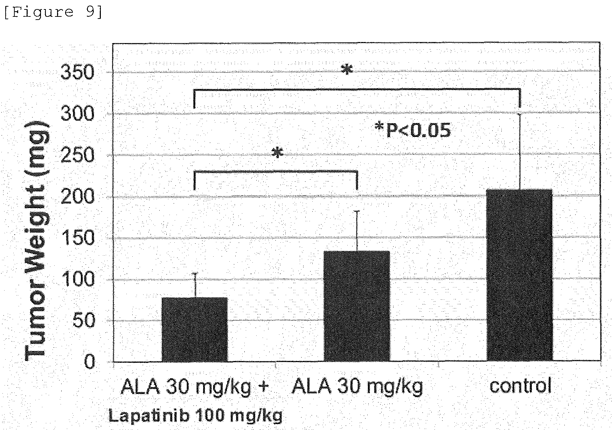

[Figure 9] Figure 9 is a view showing the results obtained by measuring the effects of ALA-PDT in the combined administration with Lapatinib, based on a difference in the weights of tumors in nude mice.

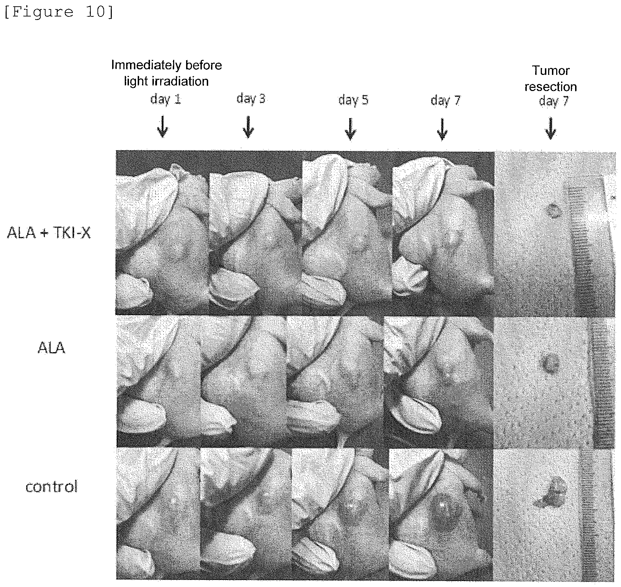

[Figure 10] Figure 10 is a view showing that the effects of ALA-PDT have been improved by the combined administration of Lapatinib in an experiment using nude mice. In the figure, the reference character "ALA+TKI-X" in the upper case indicates the results obtained by the combined administration of ALA and Lapatinib; and the reference character "ALA" in the middle case indicates the results obtained by administration of ALA alone.

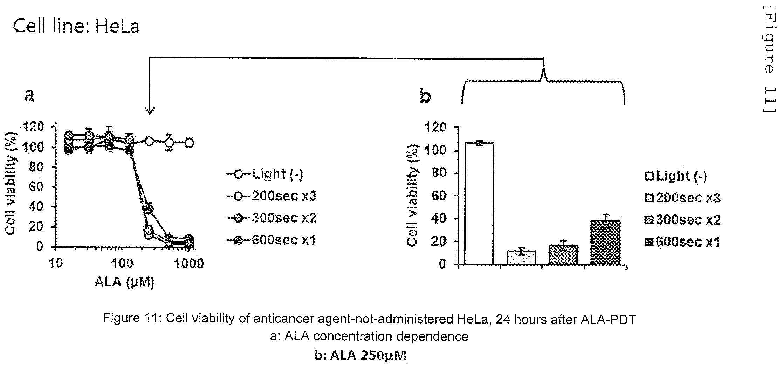

[Figure 11] Figure 11 is a view showing the cell viability of anticancer agent-not-administered HeLa, 24 hours after completion of ALA-PDT. Figure 11a shows an ALA concentration-dependent reduction in the cell viability, and Figure 11b shows extracts of the results obtained by administration of 250 µM ALA from the graph of Figure 11a.

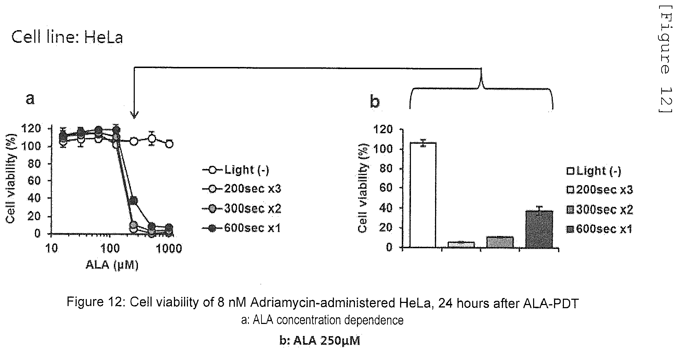

[Figure 12] Figure 12 is a view showing the cell viability of 8 nM Adriamycin-administered HeLa, 24 hours after completion of ALA-PDT. Figure 12a shows an ALA concentration-dependent reduction in the cell viability, and Figure 12b shows extracts of the results obtained by administration of 250 µM ALA from the graph of Figure 12a.

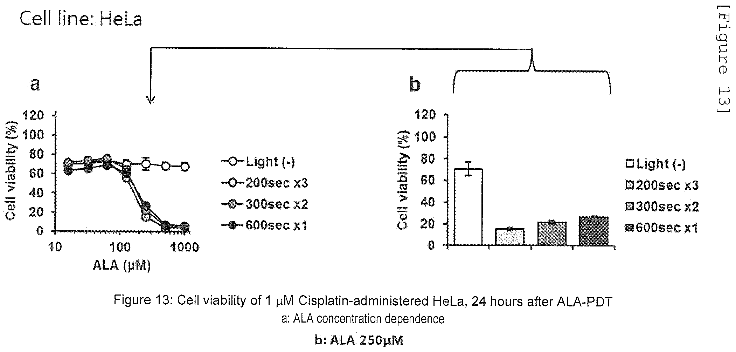

[Figure 13] Figure 13 is a view showing the cell viability of 1 µM Cisplatin-administered HeLa, 24 hours after completion of ALA-PDT. Figure 13a shows an ALA concentration-dependent reduction in the cell viability, and Figure 13b shows extracts of the results obtained by administration of 250 µM ALA from the graph of Figure 13a.

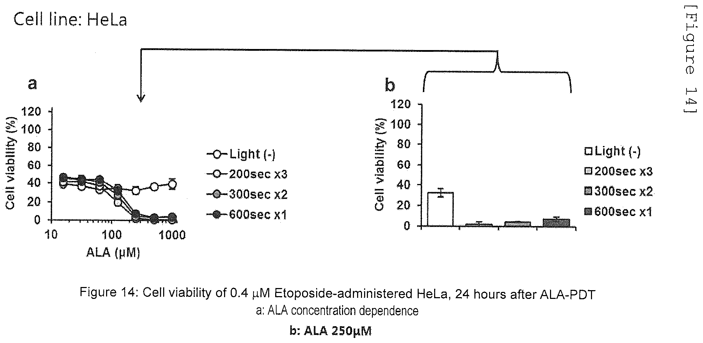

[Figure 14] Figure 14 is a view showing the cell viability of 0.4 µM Etoposide-administered HeLa, 24 hours after completion of ALA-PDT. Figure 14a shows an ALA concentration-dependent reduction in the cell viability, and Figure 14b shows extracts of the results obtained by administration of 250 µM ALA from the graph of Figure 14a.

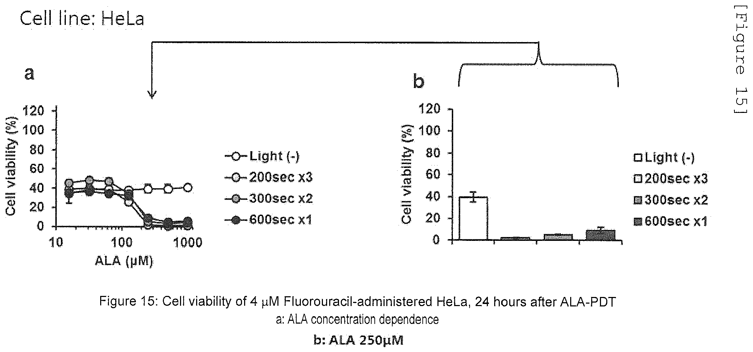

[Figure 15] Figure 15 is a view showing the cell viability of 4 µM 5-Fluorouracil-administered HeLa, 24 hours after completion of ALA-PDT. Figure 15a shows an ALA concentration-dependent reduction in the cell viability, and Figure 15b shows extracts of the results obtained by administration of 250 µM ALA from the graph of Figure 15a.

### [Description of Embodiments]

In the present description, the term "ALAs" is used to mean ALA, or a derivative thereof, or their salts.

In the present description, the term "ALA" is used to mean 5-aminolevulinic acid. ALA is also referred to as δ-aminolevulinic acid, and it is one of amino acids.

An example of the ALA derivative is a compound represented by a formula (I) as shown below. In the formula (I), R¹ represents a hydrogen atom or an acyl group, and R² represents a hydrogen atom, a linear or branched alkyl group, a cycloalkyl group, an aryl group, or an aralkyl group. It is to be noted that, in the formula (I), ALA corresponds to a case where R¹ and R² each represent a hydrogen atom.

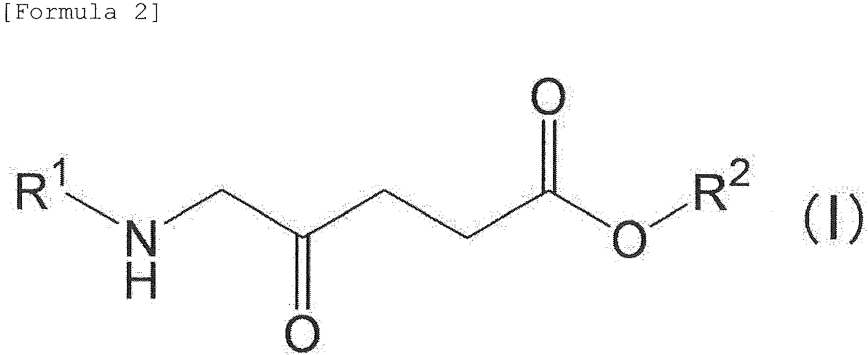

ALAs may act as an active ingredient in a living body, in the form of the ALA of the formula (I) or a derivative thereof, and such ALAs can also be administered in the form of prodrugs (precursors) that are decomposed by enzymes present in a living body.

Examples of the acyl group represented by R¹ in the formula (I) include: linear or branched alkanoyl groups having 1 to 8 carbon atoms, such as a formyl, acetyl, propionyl, butyryl, isobutyryl, valeryl, isovaleryl, pivaloyl, hexanoyl, octanoyl, or benzylcarbonyl group; and aroyl groups having 7 to 14 carbon atoms, such as a benzoyl, 1-naphthoyl, or 2-naphthoyl group.

Examples of the alkyl group represented by R² in the formula (I) include: linear or branched alkyl groups having 1 to 8 carbon atoms, such as a methyl, ethyl, propyl, isopropyl, butyl, isobutyl, sec-butyl, tert-butyl, pentyl, isopentyl, neopentyl, hexyl, heptyl, or octyl group.

Examples of the cycloalkyl group represented by R² in the formula (I) include: saturated or partially unsaturated cycloalkyl groups having 3 to 8 carbon atoms, such as a cyclopropyl, cyclobutyl, cyclopentyl, cyclohexyl, cycloheptyl, cyclooctyl, cyclododecyl, or 1-cyclohexenyl group.

Examples of the aryl group represented by R² in the formula (I) include: aryl groups having 6 to 14 carbon atoms, such as a phenyl, naphthyl, anthryl, or phenanthryl group.

Examples of the aralkyl group represented by R² in the formula (I) include groups, the aryl portions of which are the same as those of the aforementioned aryl groups, and the alkyl portions of which are the same as those of the aforementioned alkyl groups. Specific examples of such an aralkyl group include aralkyl groups having 7 to 15 carbon atoms, such as a benzyl, phenethyl, phenylpropyl, phenylbutyl, benzhydryl, trityl, naphthylmethyl, or naphthylethyl group.

A preferred ALA derivative can be a compound in which R¹ is a formyl, acetyl, propionyl, or butyryl group. Another preferred ALA derivative can be a compound in which the above R² is a methyl, ethyl, propyl, butyl, or pentyl group. A further preferred ALA derivative can be a compound in which the combination of the above R¹ and R² is (formyl and methyl), (acetyl and methyl), (propionyl and methyl), (butyryl and methyl), (formyl and ethyl), (acetyl and ethyl), (propionyl and ethyl), or (butyryl and ethyl).

Among the ALAs, examples of the salts of the ALA or a derivative thereof include pharmacologically acceptable acid-addition salts, metal salts, ammonium salts, and organic amine-addition salts. Examples of the acid-addition salts include: various inorganic acid salts such as hydrochloride, hydrobromide, hydroiodide, phosphate, nitrate, or sulfate; and various organic acid-addition salts such as formate, acetate, propionate, toluenesulfonate, succinate, oxalate, lactate, tartrate, glycolate, methanesulfonate, butyrate, valerate, citrate, fumarate, maleate, or malate. Examples of the metal salts include: various alkaline metal salts such as a lithium salt, a sodium salt, or a potassium salt; various alkaline-earth metal salts such as a magnesium salt or a calcium salt; and various metal salts such as an aluminum or zinc salt. Examples of the ammonium salts include an ammonium salt or alkylammonium salts such as a tetramethylammonium salt. Examples of the organic amine salts include various salts such as a triethylamine salt, a piperidine salt, a morpholine salt, or toluidine salt. It is to be noted that these salts can also be used in the form of a solution, upon use.

Among the aforementioned ALAs, examples of the most desirable ALA include: ALA; various esters, such as ALA methyl ester, ALA ethyl ester, ALA propyl ester, ALA butyl ester, or ALA pentyl ester; and their hydrochloride, phosphate, and sulfate. Among others, examples of particularly preferred ALA include ALA hydrochloride and ALA phosphate.

The above described ALAs can be produced according to known methods, such as chemical synthesis, production using microorganisms, and production using enzymes. In addition, the above described ALAs may form hydrates or solvates. Moreover, the ALAs can be used alone or in combination of two or more, as appropriate.

When the above described ALAs are prepared in the form of an aqueous solution, it is necessary to pay attention not to make the aqueous solution into alkaline, so as to prevent decomposition of the ALAs. If the aqueous solution becomes alkaline, the decomposition of the ALAs can be prevented by removing oxygen.

In one aspect, the preventive or therapeutic composition of the present invention may comprise a metal-containing compound, as long as such a metal-containing compound does not give unacceptable adverse effects to a living body and the like and the purpose and object of the present invention can be achieved. The metal part in such a metal-containing compound is not limited, and examples of the metal part include iron, magnesium, zinc, nickel, vanadium, cobalt, copper, chromium, and molybdenum. In light of the purpose and object of the present invention, a person skilled in the art can select a suitable dose of metal-containing compound, as appropriate, and can administer it together with ALAs.

For instance, the preventive or therapeutic composition of the present invention and a metal-containing compound can be administered, in the form of a composition comprising ALAs and a metal-containing compound, or each alone. When the present composition and the metal-containing compound are administered each alone, they may be administered simultaneously. The term "simultaneously" is used herein to mean, not only that administrations are carried out at the same time as each other, but also that although administration of the ALAs and administration of the metal-containing compound are not carried out at the same time as each other, the administrations are carried out without having a considerable interval between them, such that the administrations can give additive effects, and preferably synergic effects on a living body.

In the present description, the term "ALA-PDT" means photodynamic therapy (PDT), in which ALAs are used, and most typically means PDT in which ALA is used. In addition, the term "ALA-PDD" means photodynamic diagnosis (PDD), in which ALAs are used, and most typically means PDD in which ALA is used.

In the ALA-PDT, when PDT is carried out by administering a compound reacting with light to a subject and then applying light to a target site in the subject so as to treat the target site, ALAs that do not have photosensitization action by themselves are administered to the subject, and porphyrin (mainly, PpIX) induced via a pigment biosynthetic pathway is accumulated in cancer cells comprising therapy-resistant cancer cells, followed by excitation of the accumulated PpIX, thereby causing photoexcitation to oxygen molecules surrounding it. Singlet oxygen generated as a result of the photoexcitation exhibits a cell-killing effect because of its strong oxidizability, and the ALA-PDT utilizes this phenomenon. For the ALA-PDT, a light beam having a peak at 400 nm to 420 nm or a light beam having a peak at 600 nm to 650 nm is preferably used.

On the other hand, in the above described ALA-PDD, PpIX accumulated in cancer cells comprising therapy-resistant cancer cells is excited with a light beam to detect fluorescence, so that it can be utilized to detect or diagnose the presence or absence of cancer tissues comprising therapy-resistant cancer cells. For the ALA-PDD, a light beam having a peak at 400 nm to 420 nm is preferably used.

In the present description, the term "therapy-resistant cancer cells" means: cancer cells (resistant cancer cells), etc., which are found in advanced cancer, metastatic cancer, recurrent cancer and the like, which have subsequently acquired therapy resistance to conventional anticancer drug therapy, radiation therapy, etc.; cancer stem cells inherently having resistance to anticancer drug therapy, radiation therapy, etc., or cells that partially or entirely retain the properties of the cancer stem cells (which are also referred to as "cancer stem cell-like cells" in the present description); and lowly differentiated cancer cells, slowly-dividing cancer cells, refractory cancer cells, etc. The therapy-resistant cancer cells of the present invention are understood by a person skilled in the art as a concept of cells different from cancer cells having high sensitivity to conventional anticancer drug therapy or radiation therapy. However, when such cancer cells having high sensitivity to anticancer drug therapy or radiation therapy have become late-onset resistant cancer cells as a result of mutation, needless to say, the cancer cells are included in the therapy-resistant cancer cells of the present invention. Moreover, needless to say, in one aspect, cancer cells that are divided or proliferated from therapy-resistant cancer cells are also included in the therapy-resistant cancer cells of the present invention.

Furthermore, in the present description, the term "therapy-resistant cancer" means a cancer that comprises or is attended with the aforementioned therapy-resistant cancer cells.

When compared with normal somatic cells, cancer cells have characteristics such as high proliferation ability, cell immortalization, infiltration into peripheral tissues, and metastasis to a site remote from the cancer cells in a body. However, it has been reported that not all of cancer cells constituting cancer tissues have all of these characteristics. It has been found that only a part of the entire cancer cells actually has these characteristics, causes cancer to develop in a human or an animal, and has an ability to advance the cancer.

In the present description, the term "cancer stem cells" may indicate original cells, which typically have self-replication ability to produce completely the same cells as themselves, pluripotency of differentiating into many types of cells, and an ability to form cancer having the same phenotype as the original cancer when the cells are transplanted, and which generate a large number of cancer cells as a result of differentiation in the vicinity, while maintaining the same cells as themselves in the cancer tissue by self-replication. In the cancer stem cells, self-replication ability, pluripotency, telomerase activity, activation of anti-apoptotic pathway, and/or activation of carrier-mediated transport through a cell membrane can be typically observed.

Whether or not certain cancer cells are cancer stem cells or cancer stem cell-like cells can be determined by a person skilled in the art according to a known method. For example, the concerned cells are confirmed to be equivalent to morphologically known cancer stem cells, embryonic stem cells (ES cells) or somatic stem cells, and they can be determined to be cancer stem cells. Alternatively, the concerned cells are transplanted subcutaneously to an immunodeficient mouse and tumor tissues formed after a certain period of time has passed is analyzed, so that the cells may be determined to be cancer stem cells.

Otherwise, it is confirmed that a marker gene that is highly expressed in known cancer stem cells is expressed at a high level in the concerned cells, so that the cells may be determined to be cancer stem cells. The type of such a marker gene is not limited, and an example of the marker gene is Dclk1 (Nakanishi et al., Nature Gen. 2012.). Moreover, specifically, whether or not the concerned cells are cancer stem cells or cancer stem cell-like cells may be determined, for example, using, as an indicator, CD133⁺ (human brain tumor stem cells), CD44⁺CD24^{-/low}ESA⁺ (human breast carcinoma stem cells, ESA: epithelial specific antigen), CD44⁺ integrin α₂β₁^{hi}CD133⁺, Sca-1⁺ (human prostate carcinoma stem cells), CD133⁺ (human colon carcinoma stem cells), CD44⁺ (human head and neck squamous epithelial carcinoma stem cells), or CD44⁺CD24⁺ESA⁺ (human pancreatic carcinoma stem cells, ESA: epithelial specific antigen), although examples of the indicator are not limited thereto.

Otherwise, the expression of an undifferentiation marker, such as a marker gene specifically expressed in embryonic stem cells or somatic stem cells, is confirmed, so that the cells may be determined to be cancer stem cells. An example of the undifferentiation marker is alkaline phosphatase. It is confirmed that alkaline phosphatase staining has been tested to be positive, so that the cells can be determined to be in an undifferentiated state.

Moreover, the expression pattern of a genome-wide gene is detected by a microarray or the like, and cells having a high correlation with the expression pattern of known cancer stem cells, embryonic stem cells or somatic stem cells may be determined to be cancer stem cells.

The expression properties of a surface antigens on the cells are compared with those of known cancer stem cells, embryonic stem cells or somatic stem cells, and cells having a high correlation with them can also be determined to be cancer stem cells.

Furthermore, the methylation of DNA in the cells may be detected, and it may be then compared with that of known cancer stem cells, embryonic stem cells or somatic stem cells.

Whether or not the cells are cancer stem cells or cancer stem cell-like cells can be determined by at least one of the above described methods. Determination can also be carried out by a combination of two or more methods.

For example, when cancer cells used as targets are cultured in a low-serum or serum-free medium, if the cells have the form of a sphere type, such as the form of embryonic stem cells or somatic stem cells, it may be determined that the cells have been transformed into cancer stem cells, or at least cancer stem cell-like cells. In this case, a person skilled in the art can understand that the sphere type cells have acquired therapy resistance lately.

In the present description, the therapy-resistant cancer is preferably malignant neoplasm, but the type of the therapy-resistant cancer is not limited. Examples of the therapy-resistant cancer include primary or metastatic, invasive or non-invasive carcinomas or sarcomas, such as brain tumor, spinal cord tumor, maxillary sinus carcinoma, pancreatic ductal adenocarcinoma, carcinoma of gingiva, tongue carcinoma, lip carcinoma, nasopharyngeal carcinoma, oropharynx carcinoma, hypopharyngeal carcinoma, laryngeal carcinoma, thyroid carcinoma, parathyroid carcinoma, lung carcinoma, pleural tumor, carcinomatous peritonitis, carcinomatous pleurisy, esophageal carcinoma, stomach carcinoma, colon carcinoma, bile duct carcinoma, gallbladder carcinoma, pancreatic carcinoma, liver carcinoma, kidney carcinoma, bladder carcinoma, prostate carcinoma, penile carcinoma, testicular tumor, adrenal carcinoma, cervical carcinoma, uterine carcinoma, vaginal carcinoma, vulvar carcinoma, ovary carcinoma, bone tumor, breast carcinoma, skin carcinoma, melanoma, basal cell carcinoma, lymphoma, Hodgkin's disease, plasmacytoma, osteosarcoma, chondrosarcoma, liposarcoma, rhabdomyosarcoma, and fibrosarcoma. The therapy-resistant cancer is particularly preferably brain tumor. In addition, the therapy-resistant cancer is preferably solid cancer. When the therapy-resistant cancer cells in the present invention are cancer stem cells, the cancer stem cells may be derived from the aforementioned cancers or sarcomas.

The compound that enhances the effects of ALA-PDT or ALA-PDD may be, for example, a molecule promoting the expression of transporters (PEPT1, PEPT2, etc.) for incorporating ALA into cells, a molecule promoting the expression of ALA and porphyrin synthases (ALAS1, ALAS2, UROS, etc.), or a molecule decreasing the expression of transporters (ABCG2, ABCB6, etc.) for discharging porphyrin from cells (see Figure 5). The aforementioned compound is preferably a molecule capable of inhibiting ABCG2, although it is not limited thereto. Needless to say, examples of such a molecule are not limited to anticancer agents exhibiting therapeutic effects by the compounds alone, but also include the molecules of compounds or antibodies, which do not have anticancer action by the compounds alone but are capable of inhibiting ABCG2.

As such an ABCG2 inhibitor, those disclosed in WO2009/072267 or Int J Biochem Mol Biol 2012; 3(1): 1-27 may be used, for example. Examples of the ABCG2 inhibitor include, but are not limited to, topoisomerase II inhibitor (Mitoxantrone, Bisantrene, Etoposide, Becatecarin, NB-506, J-107088, etc.); Anthracyclines (Daunorubicin, Doxobucincin, Epirubicin, Pirarubicin, etc.);
Camptothecin analogs (topoisomerase I inhibitor) (Topotecan, SN-38, CPT-11, 9-aminocamptothecin, NX211, DX-8951f, Homocamptothecins, BN80915 (diflomotecan), Gimatecan, Belotecan, etc.);
tyrosine kinase inhibitors (in addition to the below-mentioned tyrosine kinase inhibitors, Dasatinib, Vandetanib, Nilotinib, Sorafenib, Tandutinib, CI1033 (Pan-HER TKI), CP-724,714 (HER2 TKI), Symadex (fms-like tyrosine kinase 3 inhibitor), etc.);
Antimetabolites (MTX, MTX diglutamate, MTX triglutamate (antifolate), GW1843, Tomudex (antifolates), Trimetrexatte, piritrexim, metoprine, pyrimethamine (lipophilic antifolates), 5-fluorouracil (pyrimidine analog), CdAMP (nucleotide), cladribine (nucleoside), etc.); cyclin-dependent kinase inhibitor (Flavopiridol, etc.);
CDK and aurora kinases inhibitors (JNJ-7706621, etc.); non-steroidal anti-androgens (Bicalutamide, etc.); PEITC (Phenethyl isothiocyanate, etc.); indazole-based tubulin inhibitors (TH-337, etc.); Sulfate and glucuronide conjugates of xenobiotics (Estrone 3-sulfate (E1S), 17beta-estradiol sulfate, DHEAS, 4[35S]-methylumbelliferone sulfate, E3040 sulfate, Troglitazone sulfate, 3-0-sulfate conjugate of 17alpha-ethinylestradiol, SN-38-glucuronide, [3H]17beta-estradiol-17beta-D-glucuronide, [14C]4-methylumbelliferone glucuronide, BP-3-sulfate, BP-3-glucuronide, Phenolic MPA glucuronide, etc.); Natural compounds and toxins (Folic acid, Urate, Genistein, Riboflavin (vitamin B2), plumbagin (Vitamin K3), Glutathione (GSH), Sphingosine 1-phosphate, PhIP (carcinogen), etc.); and
others ([(125)I]lodoarylazidoprazosin (IAAP), [(3)H]azidopine; Sulfasalazine (anti-inflammatory); Erythromycin (macrolide antibiotic); Ciprofloxacin, ofloxacin, norfloxacin, enrofloxacin, grepafloxacin, ulifloxacin(fluoroquinolone antibiotics); Nitrofurantoin (urinary tract antibiotic); Moxidectin (parasiticide); Albendazole suloxide and oxfendazole (anthelmintics); Ganciclovir (antiviral drug); Zidovudine, Lamivudine (NRTI); Leflunomide, A771726 (antirheumatic drugs); Diclofenac (analgesic and anti-inflammatory drug); Cimetidine (histamine H2-receptor antagonist); ME3277 (hydrophilic glycoprotein IIb/IIIa antagonist); Pitavastatin, Rosuvastatin (HMG-CoA reductase inhibitor); Dipyridamole (thromboxane synthase inhibitor); Glyburide (hypoglycemic agent); Nicardipine, nifedipine, nitrendipine (Ca2+ channel blocker); Olmesartan medoxomil (angiotensin II AT1-R antagonist); Befloxatone (selective monoamine oxidase inhibitor); Prazosin (alpha-1-adrenergic receptor antagonist); Riluzole (Na+ channels blocker); Amyloid-beta Zoledronic acid (osteotropic compound); Hesperetin conjugates, Kaempferol (see also flavonoid, WO2004/069233); an FTC (Fumitremorgin C) derivative (see Mol. Cancer Ther., 2002, 1: 417-425); estrogen and anti-estrogen (see Mol. Cancer Ther., 2003, 2: 105-112); novobiocin (see Int. J. Cancer, 2004, 108: 146-151); a diphenylacrylonitrile derivative (see WO2004/069243); an acrylonitrile derivative having a heterocyclic ring (see WO2006/106778 and WO2009/072267). As such an ABCG2 inhibitor, a molecule having anticancer action by the compound alone and being capable of inhibiting ABCG2 is preferable.

Moreover, the anticancer agent used in the present invention is not particularly limited, as long as it is an anticancer agent known to a person skilled in the art. Examples of such an anticancer agent include: alkylating agents (nitrogen mustards (cyclophosphamide, etc.); nitrosoureas, etc.); platinum compounds (cisplatin, etc.); antimetabolic agents (5-fluorouracil, etc.); antifolates (dihydropteroate synthase inhibitor; dihydrofolate reductase inhibitor, etc.); pyrimidine metabolism inhibitors; purine metabolism inhibitors; ribonucleotide reductase inhibitors; nucleotide analogs (purine analog, pyrimidine analog, etc.); topoisomerase inhibitors (type I topoisomerase inhibiting agent; type II topoisomerase inhibiting agent, etc.); microtubule inhibitors (microtubule polymerization inhibitor; microtubule depolymerization inhibitor, etc.); antitumor antibiotics; endocrine therapy; vaccine therapy; and molecularly-subjected drugs (low molecular weight pharmaceutical products (tyrosine kinase inhibiting agents; Raf kinase inhibitors; proteasome inhibiting agents, etc.); antibody drugs, etc.). These anticancer agents are considered to have an action mechanism that is fundamentally different from that of ALA-PDT. Accordingly, it can be anticipated that these anticancer agents have additive effects, and in some cases, synergic effects.

The present inventors have found that a tyrosine kinase inhibitor is able to inhibit ABCG2 associated with the excretion of PpIX in therapy-resistant cancer cells such as cancer stem cells, and thereby that the effect of ALA-PDT to inhibit the growth of therapy-resistant cancer cells and/or to kill the therapy-resistant cancer cells can be enhanced.

Accordingly, in one embodiment, the anticancer agent is preferably an anticancer agent capable of inhibiting ABCG2, and more preferably a tyrosine kinase inhibitor.

The tyrosine kinase inhibitor is not limited, but it is preferably a tyrosine kinase inhibitor against a molecule having a tyrosine residue, wherein the molecule having a tyrosine residue is selected from the group consisting of a stem cell factor receptor (KIT), an epidermal growth factor receptor (EGFR), a nerve growth factor receptor (NGFR), a colony-stimulating factor receptor (CSF-1R), a hepatocyte growth factor receptor (HGFR),a fibroblast growth factor receptor (FGFR), a vascular endothelial growth factor receptor (VEGFR), a platelet-derived growth factor receptor (PDGFR), a human epidermal growth factor receptor 2 (HER2/neu), a Src family (Src, Yes, Fyn, Fgr, Lyn, Lck, Hck, Blk, Frk), JAK, Fak, ZAP, Btk, Fps/Fes, and a Bcr-Abl.

In one aspect, the tyrosine kinase inhibitor may be a compound represented by the following formula (A), or a salt thereof: 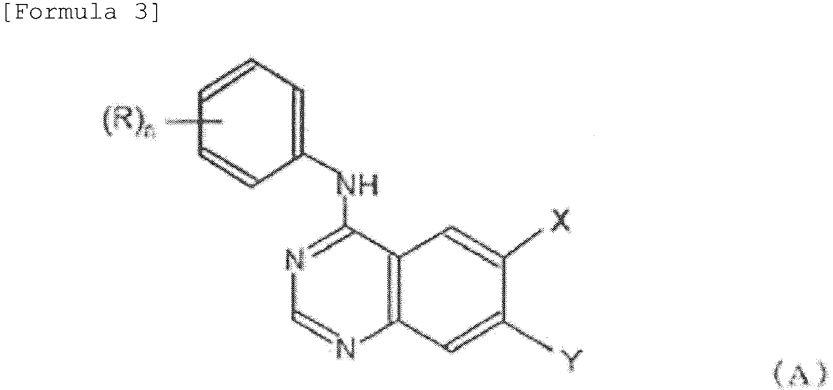 wherein R is halogen, preferably Cl or C ₂₋₅ alkynyl, more preferably ethynyl or phenyl-C₁₋₅ alkoxy, and further preferably phenylmethoxy, and R may be identical to or different from one another, wherein the phenyl may be substituted with halogen, and preferably with F; n is an integer of 1 to 5, and preferably 1 or 2; and X and Y each represent an organic group or hydrogen, and preferably 2-methoxyethoxy or 2-methylsulfonylethylamino-methyl-2-phrine, and they may be identical to or different from each other. Preferred examples of this compound include Lapatinib (n-[3-chloro-4-[(3-fluorophenyl)methoxy]phenyl]-6-[5-[(2-methylsulfonylethylamino)methyl]-2-furyl]quinazolin-4-amine), Erlotinib (N-(3-ethynylphenyl)-6,7- bis(2-methoxyethoxy)-4-quinazolinamine), and Gefitinib (N-(3-chloro-4-fluoro-phenyl)-7-methoxy-6-(3-morpholin-4-ylpropoxy)quinazolin-4-amine). Among these compounds, Lapatinib is more preferable.

The application subject of the preventive or therapeutic composition of the present invention is typically a human, and further, non-human animals such as pet animals, experimental animals and livestock animals are also included in the administration subject. In addition, in a case where it is not preferable, a human may be excluded from the subject. The above described subject may include, not only a subject which is affected with therapy-resistant cancer, but also a subject which is in danger of developing therapy-resistant cancer or having already had the therapy-resistant cancer.

The administration route of the preventive or therapeutic composition of the present invention to a subject (a living body, etc.) may be either systemic administration or local administration. Examples of the administration route include, but are not limited to, oral administration including sublingual administration; and parenteral administration, such as inhalation administration, direct administration to subject tissues or organs using a catheter, intravenous administration including infusion, transdermal administration including poultice or the like, a suppository, and administration involving force enteral nutrition methods using a nasogastric tube, nasal enteric tube, gastrostomy tube or intestinal fistula tube.

The dosage form of the preventive or therapeutic composition of the present invention is not limited, and it may be determined, as appropriate, depending on the above described administration route. Examples of the dosage form include an injection, an infusion, a tablet, a capsule, a fine granule agent, a powdered agent, a liquid agent, a water agent prepared by dissolution in syrup or the like, a poultice, and a suppository.

In order to prepare the preventive or therapeutic composition of the present invention, for example, a pharmacologically acceptable carrier, an excipient, a diluent, an additive, a disintegrator, a binder, a coating agent, a lubricant, a sliding agent, a lubricating agent, a favoring agent, a sweetener, a solubilizer, a solvent, a gelling agent, and a nutrient may be added, as necessary. Further, these additives may have influence on the absorbability and blood concentration of the preventive or therapeutic composition of the present invention, and may also provide a change in pharmacokinetics. Specific examples of the additive include water, normal saline, animal fat and oil, vegetable oil, lactose, starch, gelatin, crystalline cellulose, gum, talc, magnesium stearate, hydroxypropyl cellulose, polyalkylene glycol, polyvinyl alcohol, and glycerin.

The preventive or therapeutic medicament or the diagnostic medicament of the present invention means a combination of two or more substances or compositions, wherein the aspect of the combination is not limited.

Needless to say, to the preventive or therapeutic medicament or the diagnostic medicament according to the present invention, any given other components, such as other medicinal components, nutrients and carriers, can be added, as necessary. Examples of such any given components, which can be added to the preventive or therapeutic medicament of the present invention, include various types of mixing components for preparations, including a pharmaceutically acceptable ordinary carrier, binder, stabilizer, solvent, dispersion medium, thickener, excipient, diluent, pH buffer, disintegrator, solubilizer, solubilizing agent, and tonicity agent, such as, for example, crystalline cellulose, gelatin, lactose, starch, magnesium stearate, talc, vegetable and animal fats, oil and fat, gum, or polyalkylene glycol.

With regard to the applied dose of the preventive or therapeutic composition of the present invention, the amount of PpIX accumulated in therapy-resistant cancer cells as targets may be an effective amount in ALA-PDT, and a person skilled in the art can appropriately determine the applied dose. Naturally, conditions such as the amount applied to a subject, timing of adminsitration, administration frequency, and administration period are different, depending on the subject's age, body weight, symptoms or conditions, or the conditions of cells, tissues or organs in the subject to be prevented or treated, the ease of administration, etc.

With regard to the dose of ALAs applied to a human, in the case of oral administration for the treatment of therapy-resistant cancer for example, the applied dose is not particularly limited, as long as photosensitizing effects can be obtained from the ALAs. The applied dose may be 1 mg to 1,000 mg, preferably 5 mg to 100 mg, more preferably 10 mg to 30 mg, and further preferably 15 mg to 25 mg per kg of body weight, relative to ALA. For the aspects of treatment and administration, for example, WO2013/187069 (Title of Invention: PDT Effect Enhancing Agent) may be referred to. Moreover, it is understood that in the case of a method of preventing therapy-resistant cancer, the applied dose of ALAs is typically lower than that in a method of treating therapy-resistant cancer. For example, in the case of oral administration for the prevention of therapy-resistant cancer, the dose of ALAs applied to a human is not particularly limited, as long as photosensitizing effects can be obtained from the ALAs. The application dose may be 0.5 mg to 500 mg, preferably 2.5 mg to 50 mg, more preferably 5 mg to 15 mg, and further preferably 7.5 mg to 12.5 mg per kg of body weight, relative to ALA. Local administration would require smaller amounts of ALAs than those in the case of systemic administration.

The administration frequency of ALAs can be a single-dose or multiple-dose administration per day, or continuous administration using infusion or the like.

The administration period of ALAs can be determined by pharmacologists or clinicians in the present technical field, for example, according to known methods, based on various clinical indexes, while taking into consideration the symptoms, conditions and the like of the subject, which are to be prevented or treated.

Taking into consideration the applied dose of ALAs, timing of administration, administration frequency, administration period, etc., ALA-PDT may be performed on the therapy-resistant cancer of a subject once or several times (e.g. two times, three times, four times, or more times), so that the therapy-resistant cancer may be prevented or treated. For example, after administration of ALAs, a light beam having a specific wavelength is applied to a subject to perform PDT, and thereafter, PDT is carried out again by light beam irradiation, so that it becomes possible to kill therapy-resistant cancer cells present in a deep part of tumor, which could not be achieved by the initial PDT. Also, when ALAs are administered by multiple-dose administration, PDT may be carried out several times between the administrations of the ALAs. Moreover, after a majority of cancer cells have been killed or eliminated by conventional anticancer drug therapy or the like, ALA-PDT may be performed once or several times on the remaining (therapy resistant) cancer cells, or on tissues in which such (therapy resistant) cancer cells are likely to remain.

For example, after PDT has been performed by application of a light with a specific wavelength, in which ALAs have been used, (similar) PDT is performed again, so that it becomes possible to kill therapy-resistant cancer cells present in a deep part of tumor, which could not be achieved by the initial PDT. Furthermore, for example, conventional cancer therapy (e.g. surgical resection of tumor, anticancer drug therapy, or radiation therapy, etc.) has been first carried out to kill a majority of cancer cells, and thereafter, PDT, in which ALAs are used, is performed, so that the remaining cancer cells can be killed or the recurrence or metastasis of cancer can be prevented. In this case, the conventional cancer therapy, which has been firstly carried out, may be continuously carried out.

In a preferred aspect, upon application of ALA-PDT, an anticancer agent is combined with ALAs, and they may be then administered simultaneously or sequentially to a subject. The method of administering the ALAs may be identical to or different from the method of administering the anticancer agent. For example, the ALAs may be administered to a subject via oral administration, and the anticancer agent may be administered to the subject via intravenous administration.

Needless to say, as in the case of the ALAs, the dose of the anticancer agent applied to a subject, the timing of administration, administration frequency, administration period, etc. can be determined by a person skilled in the art, as appropriate, while taking into consideration the properties of the anticancer agent and the type thereof.

Likewise, an ABCG2 inhibitor may be used instead of the anticancer agent. In one embodiment, it is preferable that the anticancer agent also have an ABCG2-inhibitory action.

In one aspect, the ALAs comprised in the preventive or therapeutic composition of the present invention and the anticancer agent may be used, as a preventive or therapeutic medicament(s), in the form of a composition comprising the ALAs and the anticancer agent (a combination drug), or as different kits. Otherwise, they may also be administered to a subject each alone. When the ALAs and the anticancer agent are administered to a subject each alone, they may be administered simultaneously or sequentially. Herein, the term "simultaneously" is used to mean, not only that administrations are carried out at the same time as each other, but also that although administration of the ALA and administration of the anticancer agent are not carried out at the same time as each other, the administrations are carried out without having a considerable interval between them, such that the administrations can give additive effects, and preferably synergic effects.

When ALA-PDT is combined with an anticancer agent to prevent or treat a therapy-resistant cancer, for example, the anticancer agent may be administered to a subject, the ALAs may be then administered thereto, and PDT may be then carried out once or several times; or after the ALAs have been administered to a subject, the anticancer agent may be administered, and the PDT may be then carried out once or several times; or the ALAs may be administered to a subject, PDT may be then carried out thereon once or several times, and the anticancer agent may be then administered thereto. Thus, needless to say, all combinations, such as single-dose or multiple-dose administration of the ALAs, single-dose or multiple-dose administration of the anticancer agent, and single-dose or multiple-dose application of PDT, are included in the scope of the present invention.

Moreover, for example, conventional cancer therapy (e.g. surgical resection of tumor, anticancer drug therapy, or radiation therapy, etc.) has been first carried out to kill a majority of cancer cells, and thereafter, ALA-PDT is performed, so that the remaining cancer cells can be killed. In this case, the conventional cancer therapy, which has been firstly carried out, may be continuously carried out, or an anticancer agent (preferably, a tyrosine kinase inhibitor) may also be used in combination.

In another aspect, fluorescence, which is excited from PpIX that is selectively accumulated in cancer cells comprising therapy-resistant cancer cells, is detected by performing ALA-PDD using the diagnostic medicament of the present invention, so that the presence or absence of cancer tissues comprising therapy-resistant cancer cells can be determined or diagnosed. In particular, by using a tyrosine kinase inhibitor or an ABCG2 inhibitor in combination with ALAs, the excretion of PpIX from cancer cells can be decreased, and the boundary between cancer tissues and normal tissues can be clarified. Accordingly, based on the fluorescence of PpIX accumulated in the cancer tissues, the cancer tissues can be precisely diagnosed, detected, and surgically excised.

The types of therapy-resistant cancer cells and therapy-resistant cancers, which are the subjects of the diagnostic medicament of the present invention, and the types of the tyrosine kinase inhibitor and the ABCG2 inhibitor, and the like, may be the same as those described above regarding the preventive or therapeutic medicament of the present invention. For example, the therapy-resistant cancer is particularly preferably brain tumor.

Furthermore, taking into consideration the dose applied to a subject, timing of adminsitration, administration frequency, administration period, etc., a person skilled in the art can appropriately determine the use conditions for the diagnostic medicament of the present invention and can be then carried out, as with the use conditions for the preventive or therapeutic medicament of the present invention. At this time, single-dose or multiple-dose ALA administration, single-dose or multiple-dose administration of a tyrosine kinase inhibitor or an ABCG2 inhibitor, and single-dose or multiple-dose PDD application may be combined with one another, as appropriate.

In the case of the oral administration of ALAs to a human for the diagnosis of a therapy-resistant cancer for example, the dose of the ALAs applied to the human is not particularly limited, as long as the presence or absence of cancer cells can be identified by exciting ALAs-induced PpIX with a light beam and then detecting the fluorescence thereof. The applied dose may be 1 mg to 1,000 mg, preferably 5 mg to 100 mg, more preferably 10 mg to 30 mg, and further preferably 15 mg to 25 mg per kg of body weight, relative to ALA. For the aspects of diagnosis and administration, for example, WO2013/150745 (Title of Invention: Device for Determining Metastasis of Cancer to Sentinel Lymph Node) may be referred to.

Accordingly, in one aspect, the present invention may relate to a method for diagnosing a therapy-resistant cancer having therapy-resistant cancer cells in a subject. The diagnostic method comprises: administering a tyrosine kinase inhibitor or an ABCG2 inhibitor, in combination with ALAs, to a subject which is affected with or is in danger of being affected with therapy-resistant cancer, before, at the same time of, or after administration of the ALAs; then applying a light beam to a site in the subject, in which the cancer is present or is anticipated to be present, to perform PDD; and detecting fluorescence excited from PpIX selectively accumulated in cancer cells comprising therapy-resistant cancer cells, thereby determining or diagnosing whether or not the subject is affected with the therapy-resistant cancer.

### [Other effects of the present invention]

Since the expression of ABCG2 is accelerated in therapy-resistant cancer cells comprising cancer stem cells, by using an ABCG2 inhibitor (preferably, an anticancer agent capable of inhibiting ABCG2) in combination with ALAs, accumulation of PpIX in the therapy-resistant cancer cells can be promoted, and thus, the efficiency of ALA-PDT in various cancers including malignant brain tumor as a typical example (e.g. lung cancer, stomach cancer, colon cancer, breast cancer, etc.) can be advantageously improved.

Further, with regard to infiltrative cancer tissues (e.g. glioblastoma multiforme, grade IV), which is hardly excised by surgical operation, after completion of the surgical resection, a light beam is locally applied to the affected site, using an optical fiber or the like, so that cancer cells (including therapy-resistant cancer cells) that have infiltrated into normal tissues can be advantageously killed.

Still further, in the single-dose or multiple dose administration of a tyrosine kinase inhibitor capable of inhibiting the expression of ABCG2, which may be carried out several hours before application of ALA-PDT or ALA-PDD, side effects observed in long-term repetitive administration (e.g. bone marrow suppression, decreases in leukocytes, neutrophils and/or platelets, damage to the skin and liver, etc.) hardly appear. Therefore, single-dose or multiple-dose administration of a tyrosine kinase inhibitor, which is combined with ALA-PDT or ALA-PDD, has high safety, and thus,
it is advantageous.

The terms used in the present description are used to explain specific embodiments, and thus, they are not intended to limit the scope of the invention.

Moreover, the term "comprise" used in the present description is intended to mean that the mentioned matter (a member, a step, an element, a number, etc.) is present, except for a case where the term should be understood in a contextually apparently different way, and thus, the presence of other matters (other members, steps, elements, numbers, etc.) is not excluded.

Unless otherwise specified, all of the terms used herein (including technical terms and scientific terms) have the same meanings as those which are broadly understood by a person skilled in the art in the technical field, to which the present invention pertains. Unless a different definition is clearly specified, the terms used herein should be understood to have meanings which are consistent with the meanings in the present description and the relevant technical field. The terms used herein should not be understood to have ideal or excessively formal meanings.

There is a case where the embodiment of the present invention is described, while referring to a schematic view. In the case of using such a schematic view, there is a case where exaggerated expressions are used in order to make the explanation clear.

The term such as "first ..." or "second ..." is used to express various elements. It is understood, however, that these elements should not be limited by such terms. These terms are only used to distinguish one element from other elements. Thus, it is understood that it is possible to describe, for example, a first element as a second element, and likewise, to describe the second element as the first element, without deviating from the scope of the present invention, unless it includes a technical contradiction.

In the present description, when the expression "alkanoyl group containing 1 to 8 carbon atoms" is used for example, a person skilled in the art understands that this expression specifically indicates individual alkyl groups containing 1, 2, 3, 4, 5, 6, 7 or 8 carbon atoms.

In the present description, all numerical values used to indicate ingredient contents or numerical value ranges are understood to include the meaning of the term "about," unless otherwise specified.

All of the disclosures of publications cited in the present description should be incorporated herein by reference. A person skilled in the art uses and understands the relevant disclosure in those prior art publications as a part of the present description, along with the context thereof, without deviating from the gist and scope of the present invention.

Hereinafter, the present invention will be described more in detail, referring to examples. However, the present invention can be realized in various aspects, and thus, it must not be interpreted that the scope of the present invention is limited to the following examples.

### [Examples]

### < Example 1 >

### Culture of cancer stem cells

### Experimental method

Studies regarding cancer stem cells have been conducted by applying a method of concentrating and separating normal tissue stem cells. Regarding leukemic stem cells, the studies have arrived at the stage of searching for therapeutic methods targeting cancer stem cells and/or the treatment using mouse models. On the other hand, regarding solid cancer, a method of separating and concentrating the relevant cancer cells has not yet been sufficiently established. In recent years, however, it has become possible to isolate brain tumor stem cells from a patient affected with malignant brain tumor, and to culture the cells. In the present experiment, stem cell lines, which had been isolated and established from a brain tumor patient based on these methods, have been used (Hemmati HD, Nakano I, Lazareff JA, Masterman-Smith M, Geschwind DH, Bronner-Fraser M, Kornblum HI. Cancerous stem cells can arise from pediatric brain tumors. Proc Natl Acad Sci U S A. 100(25): 15178-5183, 2003.; Nakano I, Kornblum HI. Methods for analysis of brain tumor stem cell and neural stem cell self-renewal. Methods Mol Biol. 568: 37-56, 2009.).

Specifically, three types of brain tumor glioma stem cell lines (MD13, MD30, and 157NS cells), which had been isolated and established from brain tumor patients, were used, and an experiment was carried out to verify the photosensitivity of these cells to ALA-PDT. In preparation for the experiment, these cells were first cultured in a D-MEM/F12 culture solution containing 10% fetal calf serum (FCS) and in a D-MEM/F12 culture solution containing no FCS (containing L-glutamine, heparin, B27, EGF, and bFGF), and the shapes of the cells were then examined.

### Results

When the glioma stem cells were cultured in the D-MEM/F12 culture solution containing no FCS, all of the cells formed a three-dimensional mass (sphere) characteristic for stem cells. As shown in Figure 1, the diameter of the cell mass (sphere) was several hundreds of µm. On the other hand, when the cells were cultured in the D-MEM/F12 culture solution containing 10% FCS, the cells did not form such a sphere, and they have grown to a plane shape.

### < Example 2 >

### Photosensitivity of cancer stem cells to ALA-PDT

### Experimental method

Brain tumor stem cells (MD13, MD30, and 157NS cells), which had grown to a sphere type and a non-sphere type, were treated with trypsin, and thereafter, the resulting cells were each incubated at a density of 1.0 x 10⁶ cells/ml, together with 0.3 mM ALA, for 4 hours. Thereafter, a 405-nm laser light was applied to the cells. After the cells had been cultured for 7 days, the cell viability was measured according to a viable cell count measurement method (WST-8 assay) using water soluble tetrazolium salts as a coloring reagent.

### Results

Figure 2 shows the plotting of the relationship between the intensity of the laser light and the cell viability. The sphere type cells (Figure 2B) cultured in the D-MEM/F12 culture solution containing no FCS (containing L-glutamine, heparin, B27, EGF, and bFGF) had higher sensitivity to ALA-PDT, than the non-sphere type cells (Figure 2A) cultured in the D-MEM/F12 culture solution containing 10% FCS. Table 1 comprehensively shows light intensity (IC₅₀) that reduces the cell viability to 50%. It was revealed that the sphere type cells (stem cells or stem cell-like cells) had sensitivity to ALA-PDT, which was 2 times higher than that of the non-sphere type cells.

**[Table 1]**

| Table 1 Photosensitivity of cancer stem cells to ALA-PDT | | | |
|---|---|---|---|
| Cancer stem cells | IC₅₀ | | ALA-PDT sensitivity |
| | Sphere | non-sphere | |
| MD13 | 0.34 | 0.82 | 2.4 |
| MD30 | 0.83 | 1.71 | 1.9 |
| 157NS | 0.37 | 0.67 | 1.8 |

### < Example 3 >

### Photosensitivity of brain tumor cells to ALA-PDT

### Experimental method

The brain tumor cell line A172 (purchased from American Type Culture Collection) was cultured in a D-MEM/F12 culture solution containing no FCS (containing L-glutamine, heparin, B27, EGF, and bFGF). As a result, the cells formed a sphere similar to stem cells. On the other hand, when the cells were cultured in a D-MEM/F12 culture solution containing 10% FCS, the cells did not form such a sphere, and have grown to a plane shape. The cells, which had grown to a sphere type and a non-sphere type, were treated with trypsin. Subsequently, the cells were each incubated at a density of 1.0 x 10⁶ cells/ml, together with 0.3 mM ALA, for 4 hours, and thereafter, a 405-nm laser light was applied to the cells. After the cells had been further cultured for 7 days, the cell viability was measured by a WST-8 assay in the same manner as that of Example 2.

Moreover, 500 cells were collected from A172 cells, on which ALA-PDT had been performed, and the cells were then seeded on a D-MEM agar medium containing 10% FCS and an antibiotic, followed by performing a culture for 14 days. Thereafter, the cultured cells were treated with Trypan blue containing 10% formalin, so that the cell colonies were immobilized and stained. The number of colonies each consisting of 50 or more cells was counted using a stereoscopic microscope.

### Results

When the brain tumor cell line A172 was cultured in a D-MEM/F12 culture solution containing no FCS (containing L-glutamine, heparin, B27, EGF, and bFGF), the cells formed a sphere similar to stem cells (Figure 3B). In addition, the expression of CD133 and Sox-2 proteins serving as markers for brain tumor stem cells was also increased (not shown in the figure). Moreover, the photosensitivity of both sphere type and non-sphere type A172 cells to ALA-PDT was also examined in the same manner as that performed on the brain tumor stem cells MD13, MD30 and 157NS in Example 2. As a result, it was revealed that the sphere type cells had higher photosensitivity than the non-sphere type cells (Figure 3).

In the aforementioned WST-8 assay, the cell viability can be measured based on a reduction reaction using mitochondrial NAD(P)H. On the other hand, in the colony formation assay, the proliferation ability of cells can be measured. As shown in Figure 4, as a result of the examination by the colony formation assay, it was revealed that the sphere type cells had significantly higher photosensitivity than the non-sphere type cells. That is to say, it can be said that the sphere type cells (cancer stem cells) have extremely high photosensitivity, from the viewpoint of cell proliferation ability.

### < Example 4 >

### Enzymes and transporters involved in biosynthesis of porphyrin in brain tumor cells

### Experimental method

When the brain tumor cell line A172 was cultured in a D-MEM/F12 culture solution containing no FCS (containing L-glutamine, heparin, B27, EGF, and bFGF), it formed a sphere similar to stem cells, and also, its sensitivity to ALA-PDT increased (Figure 3 and Figure 4). In order to elucidate the reason, the mRNA levels of the enzyme and transporter genes involved in the biosynthesis of porphyrin were measured by quantitative PCR. Using Magna Pure LC kit (Roche), total RNA was extracted from each of the sphere type and non-sphere type A172 cells. Thereafter, using Transcriptor First-strand cDNA Synthesis Kit (Roche Diagnostics), cDNA was produced from the RNA. The quantitative PCR was carried out using Light Cycler 3 (Roche Diagnostics).

### Results

In the sphere type cells similar to cancer stem cells, the expression of transporters PEPT1 and PEPT2, which are associated with incorporation of ALA into the cells, and the expression of porphyrin synthase and an ABCB6 gene, were accelerated, in comparison to the non-sphere type cells (Figure 5). In addition, regarding the intracellular PpIX level as well, the PpIX level in the sphere type cells became two times higher than that in the non-sphere type cells (not shown in the figure).

### < Example 5 >

### Inhibition of transport function of ABCG2 by tyrosine kinase inhibitor

### Experimental method

Forty-eight types of ABC transporter genes are encoded in the human genomic DNA. Among these genes, ABCG2 has an ability to physiologically transport intracellular PpIX to the outside of cells.

Moreover, it has been reported that the expression of ABCG2 is significantly high in stem cells, and that ABCG2 actively discharges Hoechst 33342, which is used to isolate stem cells or SP (side population) cells by a flow cytometric method, from the cells (Zhou S, Schuetz JD, Bunting KD, Colapietro AM, Sampath J, Morris JJ, Lagutina I, Grosveld GC, Osawa M, Nakauchi H, Sorrentino BP. The ABC transporter Bcrp1/ABCG2 is expressed in a wide variety of stem cells and is a molecular determinant of the side-population phenotype. Nat Med. 7(9): 1028-1034, 2001.).

To date, the present inventors have developed a high performance screening system for searching for a compound and a drug that inhibit the transport function of ABCG2, and have searched for an efficient inhibitor molecule (Saito H, Hirano H, Nakagawa H, Fukami T, Oosumi K, Murakami K, Kimura H, Kouchi T, Konomi M, Tao E, Tsujikawa N, Tarui S, Nagakura M, Osumi M and Ishikawa T. A new strategy of high-speed screening and quantitative structure-activity relationship analysis to evaluate human ATP-binding cassette transporter ABCG2-drug interactions. J. Pharmacol. Exp. Ther. 317(3), 1114-1124, 2006.). As a result, the inventors have discovered from an experiment that a tyrosine kinase inhibitor, Gefitinib, effectively inhibits ABCG2 (An R, Hagiya Y, Tamura A, Li S, Saito H, Tokushima D, and Ishikawa T. Cellular phototoxicity evoked through the inhibition of human ABC transporter ABCG2 by cyclin-dependent kinase inhibitors in vitro. Pharm Res. 26(2), 449-458, 2009.).

In the present experiment, using Hoechst 33342 and a plurality of tyrosine kinase inhibitors, an experiment was carried out to screen for an inhibitor of ABCG2.

Specifically, using Erlotinib, Gefitinib, Lapatinib, Imatinib, Nilotinib and Dasatinib as tyrosine kinase inhibitors, and also using an L-type calcium channel inhibitor, Verapamil, as a control, screening was carried out for inhibition of the transport function of ABCG2.

Flp-In-293 cells (Flp-In-293/ABCG2 cells), which express human ABCG2, were cultured together with Hoechst 33342 as a substrate of ABCG2 and the aforementioned tyrosine kinase inhibitors for a certain period of time. Thereafter, the level of Hoechst 33342 accumulated in individual cells was measured using FACS (Beckman Coulter). In order to demonstrate that it was inhibition specific to ABCG2, the same experiment as described above was also carried out on Flp-In-293/Mock cells (which have not expressed ABCG2). Specific experimental procedures are as follows.

Flp-In-293/Mock and Flp-In-293/ABCG2 were each washed with PBS (phosphate buffered saline) twice, and were then peeled using Trypsin-EDTA. 10⁶ cells were suspended in a DMEM medium (2% FBS). Subsequently, 5 µg/mL Hoechst 33342 (Invitrogen) alone was added to the suspension, or 5 µg/mL Hoechst 33342 was combined with various types of inhibitors in concentrations of 1 nM to 100 µM, and the combination was then added to the suspension. The obtained mixture was cultured at 37°C for 90 minutes. After completion of the culture, the cultured cells were washed with cold PBS (2% FBS), and were then suspended again in cold PBS (2% FBS). Thereafter, intracellular fluorescence intensity was measured using FACS (Beckman Coulter). 50,000 cells were measured for a single measurement. Hoechst 33342 accumulated in the cells was excited with UV laser, the fluorescence at 675 nm was then detected, and the mean fluorescence intensity (MFI) of the fluorescence of the measured cells was then calculated. Based on the obtained results, the inhibitory percentage against ABCG2 was calculated.

### Results

The MFI values of Flp-In-293/Mock cells and Flp-In-293/ABCG2 cells upon addition of Hoechst 33342 alone were set at 100% and 0%, respectively. The MFI values of individual addition groups are shown in Figure 6. The longitudinal axis indicates the inhibitory percentage of each tyrosine kinase inhibitor to inhibit the excretion of Hoechst 33342 (which reflects inhibition of ABCG2 function), whereas the horizontal axis indicates the concentration of each tyrosine kinase inhibitor. From these results, it was revealed that, among the tested tyrosine kinase inhibitors, Lapatinib inhibits ABCG2 most strongly, and Erlotinib and Gefitinib have ABCG2-inhibitory ability almost equivalent to each other.

### < Example 6 >

### Inhibition of ABCG2 transport and intracellular accumulation of protoporphyrin IX (PpIX) biosynthesized from ALA, by Lapatinib

### Experimental method

Human glioblastoma astrocytoma U87MG cells were cultured together with 2 mM ALA and Lapatinib in various concentrations for 4 hours, and thereafter, the fluorescence intensity (MFI value) of PpIX, which was biosynthesized from ALA and was accumulated in the cells during the culture, was measured using FACS (Beckman Coulter). Specific experimental procedures are as follows.

U87MG cells were washed with PBS twice, and were then peeled using Trypsin-EDTA. 10⁶ cells were suspended in a low-serum DMEM medium (2% FBS). Subsequently, 2 mM ALA alone was added to the suspension, or 2 mM ALA was combined with Lapatinib or Gefitinib in a concentration from 1 nM to 100 µM, and the combination was then added to the suspension. Thereafter, the obtained mixture was cultured at 37°C for 4 hours. After completion of the culture, the cultured cells were washed with cold PBS (2% FBS), and were then suspended again in cold PBS (2% FBS). Thereafter, intracellular fluorescence intensity was measured using FACS (Beckman Coulter). 50,000 cells were measured for a single measurement. PpIX accumulated in the cells was excited with UV laser (325 nm), the fluorescence at 575 nm was then detected, and the mean fluorescence intensity (MFI) of the fluorescence of the measured cells was then calculated. Based on the obtained results, intracellular accumulation of PpIX was relatively calculated, and inhibition of the PpIX transport of ABCG2 by Lapatinib or Gefitinib was estimated.

### Results

Human glioblastoma astrocytoma U87MG cells biosynthesize PpIX as a result of culture with ALA. When the human glioblastoma astrocytoma U87MG cells were cultured together with 2 mM ALA and Lapatinib in various concentrations for 4 hours, the fluorescence intensity of PpIX accumulated in the cells increased. An increase in the fluorescence intensity of PpIX depended on the concentration of Lapatinib, and the fluorescence intensity of PpIX significantly increased when Lapatinib had a concentration from 1 to 100 µM. It was revealed that Lapatinib significantly inhibited ABCG2 in the concentration region of 10 nM or more, and that PpIX biosynthesized from ALA was thereby accumulated in the cells. On the other hand, even when ABCG2 was inhibited by Gefinitib, intracellular accumulation of PpIX was observed, but the amount of PpIX accumulated was not as large as that in the case of inhibition by Lapatinib (Figure 7). This is considered because Gefitinib has an ABCG2-inhibitory activity that is weaker than that of Lapatinib and this would be reflected in the results (see Figure 6), and also because of a difference in culture conditions and the like.

### < Example 7 >

### Inhibition of ABCG2 transport by Lapatinib and effects of ALA-PDT: Experiment using cultured cells

### Experimental method

Human glioblastoma astrocytoma U87MG cells (10⁶ cells) were suspended in DMEM (2% FBS), and thereafter, 10 mM ALA alone was added to the suspension, or 10 mM ALA was combined with Lapatinib in a concentration from 1 nM to 300 µM, and the combination was then added to the suspension. The obtained mixture was cultured at 37°C for 4 hours. Thereafter, the cells were irradiated with LED having a peak at 635 nm (12 J/cm²), and the cell viability was then measured by an MTT method. It is to be noted that the MTT method has the same measurement principle as that of the WST-8 assay, and that this is a colorimetric determination method for measuring an enzymatic activity of reducing MTT to a formazan pigment (violet).

### Results

By adding ALA alone to human glioblastoma astrocytoma U87MG cells, then culturing the mixture, and then applying LED to the resulting cells (12 J/cm²), approximately 50% of the cells died (corresponding to a case where, in Figure 8, the concentration of Lapatinib is 0).

On the other hand, by culturing the U87MG cells together with 10 mM ALA and Lapatinib in various concentrations, the sensitivity of the cells to LED irradiation (12 J/cm²) was increased in a concentration-dependent manner (wherein the cells were easily killed). These results demonstrate that PpIX biosynthesized from ALA was accumulated in the cells as a result of the inhibition of ABCG2 by Lapatinib, and that the sensitivity of the cells to light irradiation was increased (Figure 8).

### < Example 8 >

### Inhibition of ABCG2 transport by Lapatinib and effects of ALA-PDT: Experiment using nude mice

### Experimental method

Human glioblastoma astrocytoma U87MG cells were cultured in a 10% FCS-containing D-MEM medium (Sigma-Aldrich), and the number of surviving cells was then counted using Trypan blue. Subsequently, U87MG cells (5 x 10⁶ cells/0.1 ml/mouse) were transplanted subcutaneously into the dorsal portion of BALB/c-nu/nu nude mice via injection. Three days later, ALA alone (30 mg/kg body weight, p.o.), or a combination of ALA (30 mg/kg body weight, p.o.) with Lapatinib (100 mg/kg body weight, p.o.), was administered to the nude mice via oral administration. Then, three hours after the administration, LED having a luminescence peak at 635 nm was applied to the tumor-transplanted portion. One week after the LED irradiation, the tumors were excised from the mice, and the weights thereof were then measured.

### Results

In the case of the mice, to which ALA-PDT was carried out by administration of a combination of ALA (30 mg/kg body weight, p.o.) with Lapatinib (100 mg/kg body weight, p.o.) via oral administration, the growth of the tumor was significantly suppressed, in comparison to control mice and the mice in the single ALA (30 mg/kg body weight, p.o.) administration group (Figure 9 and Figure 10).

### < Example 9 >

### Effects of multiple-dose PDT application

### Experimental method

HeLa cells (human-derived uterine cervix cancer cell line; 1 x 10⁵ cells) were seeded on a 60-mm dish, and then cultured for 24 hours. Thereafter, an anticancer agent was administered to the culture solution. The types of the administered anticancer agents and the concentrations of the anticancer agents in the culture solution are shown in Table 2. Seventy-two hours after administration of the anticancer agent, the cells were recovered by a trypsin-EDTA treatment, and using an aliquot of the recovered cells, the number of surviving cells was confirmed by Trypan blue staining. The surviving cells (5 x 10³ cells) were seeded on a 96-well plate. In the present example, these cells surviving 72 hours after administration of the anticancer agent were used as therapy-resistant cancer.

**[Table 2]**

| Table 2 Used anticancer agents and concentrations thereof | | | | |
|---|---|---|---|---|
| Anticancer agent | Adriamycin | Cisplatin | Etoposide | 5-Fluorouracil |
| Concentration | 8nM | 1µM | 0.4µM | 4µM |

Twenty-four hours after the seeding of the surviving cells on the 96-well plate, 5-aminolevulinic acid hydrochloride (ALA) was administered to the cells, and the resulting cells were then cultured for 4 hours. Thereafter, 631-nm LED light was applied to the cells to a total dose of 60 J/cm², one to three times. In the case of multiple-dose light irradiation, the interval between light irradiations was set at 2 hours. Twenty-four hours after the light irradiation, the resulting cells were washed with PBS twice, and the cell viability was then measured by the WST8 assay. It is to be noted that PDT was carried out with reference to the method described in Photodiagnosis Photodyn Ther. 9 (3): 204-14 (2012).

### Results

A change in the cell viability, 24 hours after application of ALA-PDT to each of anticancer agent-not-administered HeLa cells and therapy-resistant cancer HeLa cells, is shown in each of Figures 11 to 15. The horizontal axis in each of Figure 11a, Figure 12a, Figure 13a, Figure 14a and Figure 15a indicates the concentration of ALA administered. Figure 11b, Figure 12b, Figure 13b, Figure 14b, and Figure 15b each show extracts of the results obtained by administration of 250 µM ALA from the graphs shown in Figure 11a, Figure 12a, Figure 13a, Figure 14a, and Figure 15a, respectively. The longitudinal axis in each figure indicates the percentage of cell viability in the experiment groups, while the cell viability is set at 100% when the experiment was carried out under the same conditions as those described above with the exception that ALA was not administered.

In all cases of anticancer agent-not-administered HeLa cells and Hela cells that have survived after administration of various types of anticancer agents (therapy-resistant cancer), the cell viability of the HeLa cells was decreased, when ALA in a concentration of 125 µM or higher was administered. Moreover, 125 to 250 µM ALA was administered to the cells, the cell viability was decreased depending on the number of light irradiations. From these results, it was found that, when light irradiation was carried out using a light with identical irradiation energy, multiple-dose ALA-PDT application has higher therapeutic effects than single-dose ALA-PDT application.

## Claims

1. A preventive or therapeutic composition for a therapy-resistant cancer having therapy-resistant cancer cells used in photodynamic therapy, comprising a compound represented by the following formula (I), or a salt thereof: 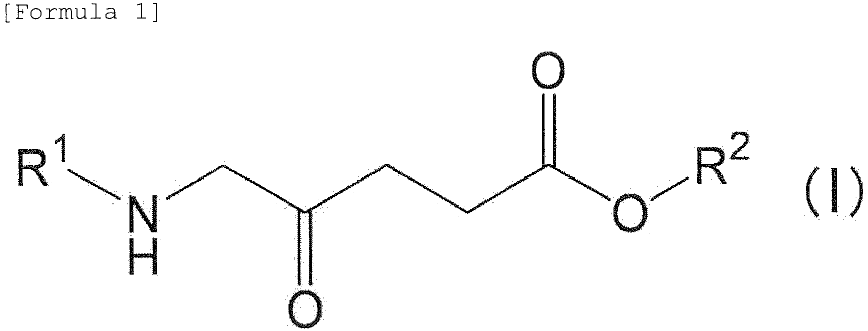 wherein R¹ represents a hydrogen atom or an acyl group, and R² represents a hydrogen atom, a linear or branched alkyl group, a cycloalkyl group, an aryl group, or an aralkyl group.

2. The preventive or therapeutic composition according to claim 1, wherein the therapy-resistant cancer cells comprise cancer stem cells.

3. The preventive or therapeutic composition according to claim 1 or 2, wherein the cancer stem cells are brain tumor stem cells.

4. The preventive or therapeutic composition according to any one of claims 1 to 3, wherein the photodynamic therapy is carried out on therapy-resistant cancer cells in a subject two or more times.

5. A preventive or therapeutic medicament for a therapy-resistant cancer, comprising a combination of (1) the preventive or therapeutic composition according to any one of claims 1 to 4 and (2) an anticancer agent administered simultaneously or sequentially.

6. The preventive or therapeutic medicament according to claim 5, wherein the anticancer agent is a tyrosine kinase inhibitor.

7. The preventive or therapeutic medicament according to claim 6, wherein
the tyrosine kinase inhibitor is a tyrosine kinase inhibitor acting against a molecule having a tyrosine residue, and
the molecule having a tyrosine residue is selected from the group consisting of a stem cell factor receptor (KIT), an epidermal growth factor receptor (EGFR), a nerve growth factor receptor (NGFR), a colony-stimulating factor receptor (CSF-1R), a hepatocyte growth factor receptor (HGFR), a fibroblast growth factor receptor (FGFR), a vascular endothelial growth factor receptor (VEGFR), a platelet-derived growth factor receptor (PDGFR), a human epidermal growth factor receptor 2 (HER2/neu), a Src family, JAK, Fak, ZAP, Btk, Fps/Fes, and Bcr-Abl.

8. The preventive or therapeutic medicament according to claim 6 or 7, wherein the tyrosine kinase inhibitor is an ABCG2 inhibitor.

9. The preventive or therapeutic medicament according to any one of claims 5 to 8, wherein the combination aspect is a combination drug or a kit.

10. The preventive or therapeutic composition according to any one of claims 1 to 4, used in combination simultaneously or sequentially with an anticancer agent.

11. A combination of (1) the preventive or therapeutic composition according to any one of claims 1 to 4 and (2) an anticancer agent for treating a therapy-resistant cancer, wherein
(1) the therapeutic composition and (2) the anticancer agent are administered simultaneously or sequentially.

12. A method for preventing or treating a therapy-resistant cancer having therapy-resistant cancer cells in a subject, comprising:
(I) a step of administering to a subject affected with or in danger of being affected with a therapy-resistant cancer a compound represented by the following formula (I) or a salt thereof: 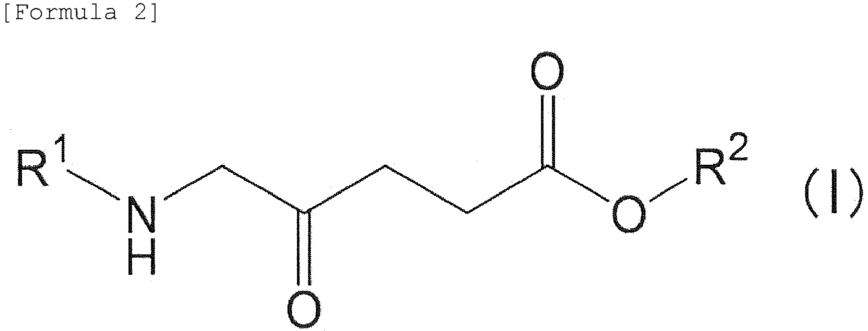 wherein R¹ represents a hydrogen atom or an acyl group, and R² represents a hydrogen atom, a linear or branched alkyl group, a cycloalkyl group, an aryl group, or an aralkyl group; and
(II) a step of performing photodynamic therapy on the cancer cells in the subject.

13. The preventive or therapeutic method according to claim 12, wherein
in the step (II), the photodynamic therapy is performed on the therapy-resistant cancer cells two or more times.

14. The preventive or therapeutic method according to claim 12 or 13, further comprising a step of administering an anticancer agent simultaneously or sequentially to the subject, before, during, or after the step (I) or (II).

15. Use of a compound represented by the following formula (I), or a salt thereof, for production of a preventive or therapeutic medicament for a therapy-resistant cancer used in photodynamic therapy: 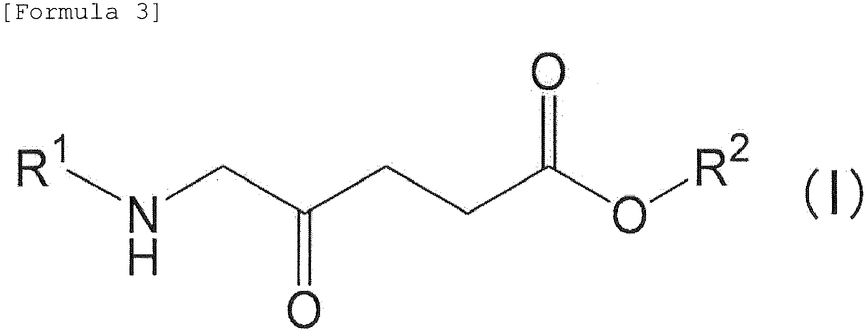 wherein R¹ represents a hydrogen atom or an acyl group, and R² represents a hydrogen atom, a linear or branched alkyl group, a cycloalkyl group, an aryl group, or an aralkyl group.

16. A medicament for diagnosing a therapy-resistant cancer having therapy-resistant cancer cells in photodynamic therapy, comprising a combination of
(A) a compound represented by the following formula (I), or a salt thereof: 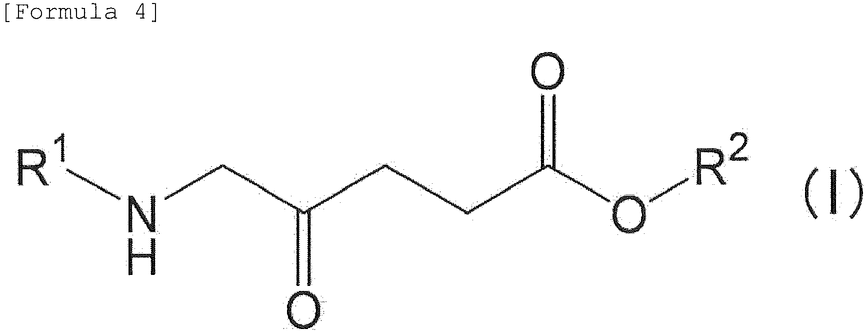 wherein R¹ represents a hydrogen atom or an acyl group, and R² represents a hydrogen atom, a linear or branched alkyl group, a cycloalkyl group, an aryl group, or an aralkyl group, and
(B) a tyrosine kinase inhibitor or an ABCG2 inhibitor, administered simultaneously or sequentially.
